# EUROPEAN PATENT SPECIFICATION

(11) **EP 1 829 960 B1**
(45) Date of publication and mention of the grant of the patent: **14.09.2011**
(21) Application number: 05816539.0
(22) Date of filing: 08.12.2005
(51) Int. Cl.: C12N 5/10, A01H 5/00, C12N 15/09

(54) **METHOD FOR PRODUCTION OF PLANT CELL HAVING CHROMOSOME LOSS**
VERFAHREN ZUR HERSTELLUNG EINER PFLANZENZELLE MIT CHROMOSOMENVERLUST
PROCEDE POUR LA PRODUCTION DE CELLULE VEGETALE PRESENTANT UNE PERTE DE CHROMOSOMES

(30) Priority: 08.12.2004 JP 2004382394; 18.05.2005 JP 2005174225
(43) Date of publication of application: 05.09.2007
(73) Proprietor: NIPPON PAPER INDUSTRIES CO., LTD., Kita-ku, Tokyo 114-0002 (JP)
(72) Inventor: NANTO, Kazuya, c/o Nippon Paper Industries Co.,Ltd., Tokyo 114-0002 (JP); EBINUMA, Hiroyasu, c/o Nippon Paper Inustries Co.,Ltd., Tokyo 114-0002 (JP)
(74) Representative: Ward, David Ian
(86) International application number: PCT/JP2005/023184
(87) International publication number: WO 2006/062259

(56) References cited:
- WO-A-00/55325
- JP-A- 2004 166 654
- JP-A- 2004 321 187
- KAWASAKI HIDEKI ET AL: "A DNA construct useful for specific chromosome loss in Saccharomyces cerevisiae" JOURNAL OF FERMENTATION AND BIOENGINEERING, vol. 77, no. 2, 1994, pages 125-130, XP002467355 ISSN: 0922-338X
- QIN M ET AL: "CRE RECOMBINASE-MEDIATED SITE-SPECIFIC RECOMBINATION BETWEEN PLANT CHROMOSOMES" PROCEEDINGS OF THE NATIONAL ACADEMY OF SCIENCES OF USA, NATIONAL ACADEMY OF SCIENCE, WASHINGTON, DC, US, vol. 91, no. 5, 1 March 1994 (1994-03-01), pages 1706-1710, XP000615549 ISSN: 0027-8424
- KOEBNER ROBERT ET AL: "Large-scale mutagenesis directed at specific chromosomes in wheat" GENOME, vol. 44, no. 1, February 2001 (2001-02), pages 45-49, XP002467356 ISSN: 0831-2796
- WIDIANTO D. ET AL: 'A method for fusing chromosomes in Saccharomyces cerevisiae' JOURNAL OF FERMENTATION AAND BIOENGINEERING vol. 83, no. 2, 1997, pages 125 - 131, XP003001135
- GILBERTSON L. ET AL: 'Cre-lox recombination: Cre-ative tools for plant biotechnology' TRENDS IN BIOTECHNOLOGY vol. 21, no. 12, 2003, pages 550 - 555, XP004473520
- MEDBERRY S.L. ET AL: 'Intra-chromosomal rearrangements generated by Cre-lox site-specific recombination' NUCLEIC ACIDS RES. vol. 23, no. 3, 1995, pages 485 - 490, XP003001136
- HOESS R.H. ET AL: 'The role of the loxP spacer region in P1 site-specific recombination' NUCLEIC ACIDS RES. vol. 14, no. 5, 1986, pages 2287 - 2300, XP002918423
- MATSUZAKI H. ET AL: 'Chromosome engineering in Saccharomyces cerevisiae by using a site-specific recombination system of a yeast plasmid' J. BACTERIOL. vol. 172, no. 2, 1990, pages 610 - 618, XP002940365

## Description

### TECHNICAL FIELD

The present invention relates to a production process of a plant cell in which one or more chromosomes are disappeared.

### BACKGROUND ART

In multicellular organisms, the chromosome number in somatic cells can usually be expressed as 2n. In response to these normal organisms, somatic cells and individual organisms with their chromosome numbers altered are referred to as aneuploid cells or aneuploid individual organisms, or simply aneuploids, which are extremely important in breeding and genetics.

Specifically, most of these aneuploids indicate characteristics different from those of normal organisms and thus naturally-occurring or artificially-produced aneuploids of vegetables, flowers and ornamental plants and others are used as materials for further breeding, or are used as new varieties in themselves.

Among the aneuploids, in monosomics with only one chromosome disappeared, a gene locus on the disappeared chromosome does not indicate a normal segregation ratio in F1 generation, obtained by autologous hybridization, and thus, by using this, the gene locus can be determined. This technique is called monosomic analysis; for example, a series of monosomics covering all 21 pairs of chromosomes of bread wheat (Chinese Spring) was produced, and gene loci associated with many characteristics were determined through studies on characteristics of these monosomics and their self-fertilized F1 generations (Non-patent document 1).

However, generation of aneuploids is very difficult. For example, the monosomic series of bread wheat was produced using the haploids obtained by crossbreeding rye and bread wheat; however, species whose monosomics can be obtained by this technique is extremely limited. On the other hand, aneuploids such as monosomics can also be obtained by exposing cells of the organisms to chemical substances such as colchicine and EMS (ethylmethane sulphonate) or radiation (Non-patent document 2); however, use of these methods requires advanced techniques and often produces unexpected damage to other chromosomes which are not to be disappeared.
Non-patent document 1: Sears, Univ. Missouri Res. Bull. (US), 572: 1-58 (1954)
Non-patent document 2: Yasuo Ukai, Plant Breeding, pp. 259-261 (2003)

### DISCLOSURE OF INVENTION

The inventors performed a keen examination regarding the task of providing a simple aneuploid production process, applicable to all species, without producing unexpected damage to other chromosomes which were not to be disappeared, and as a result, found that this could be resolved by use of a site-specific recombination system and achieved the present invention.

Specifically, the present invention relates to the following (1)-(14):
(1) A process for producing a plant cell in which one or more chromosomes are disappeared, said process comprises the following steps (A)-(C) (hereinafter, referred to as the first embodiment of the present invention):
   (A) introducing a vector comprising
      two site-specific recombinase recognition sequences oriented in the opposite direction,
      a site-specific recombinase gene which is positioned inside or outside of the region between the two site-specific recombinase recognition sequences and which encodes a site-specific recombinase which recognizes the two site-specific recombinase recognition sequences, and a negative selectable marker gene, positioned inside or outside of the region between the two site-specific recombinase recognition sequences oriented in the opposite direction
      into a plant cell;
   (B) culturing and growing the vector-introduced plant cell (A); and
   (C) selecting a cell in which a chromosome is disappeared, from the cell grown in (B),
      wherein the plant cell is cultured under conditions where the negative selectable marker gene functions in the step (B).
(2) A process for producing a plant cell in which one or more chromosomes are disappeared, said process comprising the following steps (A)-(C) (hereinafter, referred to as the second embodiment of the present invention):
   (A) introducing a vector comprising
      one site-specific recombinase recognition sequence comprising a point-symmetric nucleotide sequence,
      a site-specific recombinase gene encoding a site-specific recombinase which recognizes the site-specific recombinase recognition sequence, and a negative selectable marker gene
      into a plant cell;
   (B) culturing and growing the vector-introduced plant cell in (A); and
   (C) selecting a cell in which a chromosome is disappeared, from the cell grown in (B),
      wherein a plant cell is cultured under conditions where the negative selectable marker gene functions in the step (B).
(3) A process for producing a plant cell in which one or more chromosomes are disappeared, said process comprises the following steps (A)-(D) (hereinafter, referred to as the third embodiment of the present invention):
   (A) introducing a vector comprising two site-specific recombinase recognition sequences oriented in the opposite direction, and a negative selectable marker gene positioned inside or outside of the region between the two site-specific recombinase recognition sequences, or a vector comprising one site-specific recombinase recognition sequence comprising a point-symmetric nucleotide sequence, and a negative selectable marker gene into a plant cell;
   (B) culturing the vector-introduced plant cell in (A) and allowing the site-specific recombinase which recognizes the two site-specific recombinase recognition sequences to act transiently in the cell during at least growth of the cell;
   (C) culturing and growing a cell in which the site-specific recombinase has been allowed to act in (B); and
   (D) selecting a cell in which a chromosome is disappeared, from the cell grown in (C),
      wherein the plant cell is cultured under conditions where the negative selectable marker gene functions in the step (C).
(4) The process for producing a plant cell in which one or more chromosomes are disappeared according to the above (3), wherein the site-specific recombinase is allowed to act transiently by introducing the site-specific recombinase into the cultured plant cell in the step (B).
(5) The process for producing a plant cell in which one or more chromosomes are disappeared according to the above (3), wherein the site-specific recombinase is allowed to act transiently by introducing a site-specific recombinase gene encoding the site-specific recombinase into the cultured plant cell in the step (B).
(6) The process for producing a plant cell in which one or more chromosomes are disappeared according to the above (5), wherein introduction of the site-specific recombinase gene is carried out by introducing a vector comprising a site-specific recombinase gene and a negative selectable marker gene, into a plant cell
(7) The process for producing a plant cell in which one or more chromosomes are disappeared according to the above (5),
   wherein the site-specific recombinase gene is positioned between the two site-specific recombinase recognition sequences, oriented in the same direction, which are recognized by the site-specific recombinase encoded by the site-specific recombinase gene, in a vector, and
   the vector is introduced into a plant cell.
(8) The process for producing a plant cell in which one or more chromosomes are disappeared according to the above (7), wherein a negative selectable marker gene, together with a site-specific recombinase gene, is positioned between the two site-specific recombinase recognition sequences, oriented in the same direction, in the vector.
(9) The process for producing a plant cell in which one or more chromosomes are disappeared according to the above (1), (2), (3), (6) or (8), wherein a lethal induction gene or a morphological abnormality induction gene is used as the negative selectable marker gene.

According to the present invention, plant cells in which one or more chromosomes are disappeared can be produced regardless of species.

In order to produce them, it is sufficient to use only very general techniques as a gene transfer technique for a plant or a plant tissue culture technique. The vector used here for gene introduction has a very simple construction.

In the present invention, a plant individual can be reproduced from plant cells in which one or more chromosomes are disappeared without proceeding through reproductive cells.

Thus, the present invention can be applied to all plant species, and provides a simple production process of monosomics such as aneuploids like, i.e., aneuploid plant cells or plant individual, which are important in breeding or genetics.

In addition, generation of the aneuploids never causes unexpected damage to chromosomes other than ones which are to be disappeared.

### BRIEF DESCRIPTION OF THE DRAWINGS

Fig. 1 shows the mechanism of chromosome disappearance when two recognition sequences oriented in the opposite direction exist on the chromosome.
Fig. 2 shows the mechanism of chromosome disappearance when one recognition sequence containing a point-symmetric nucleotide sequence exists on the chromosome.
Fig. 3 shows the mechanism of chromosome disappearance in plant cells by the first embodiment of the present invention.
Fig. 4 shows the mechanism of chromosome disappearance in plant cells by the second embodiment of the present invention.
Fig. 5 shows the mechanism of chromosome disappearance in plant cells by the third embodiment of the present invention.
Fig. 6 shows the mechanism of chromosome disappearance by the functions of a free recombinase gene in the third embodiment of the present invention.
Fig. 7 shows the mechanism of chromosome disappearance in plant cells when a vector comprising a negative selectable marker gene, together with two recognition sequences and a recombinase gene, is used in the first embodiment of the present invention.
Fig. 8 shows the functions of a negative selectable marker gene when a vector comprising a negative selectable marker gene, together with a recombinase gene, is used as a vector for introduction of a recombinase gene in the third embodiment of the present invention.
Fig. 9 shows the functions of a negative selectable marker gene when a vector comprising a negative selectable marker gene, together with two recognition sequences and a recombinase gene, is used as a vector for introduction of a recombinase gene in the third embodiment of the present invention.
Fig. 10 shows a construction scheme of pTSspsRScodN, a vector for introduction of a recognition sequence.
Fig. 11 shows a construction scheme of pTShygMRS36RRubipt, a vector for introduction of a recombinase gene.
Fig. 12 shows the mechanism of chromosome disappearance in Example 1 of the present invention.
Fig. 13 shows a positional relationship between the primer used in Example 1 of the present invention and a chromosome to which pTSspsRScodN was introduced or a chromosome from which the region between two recognition sequences is disappeared after introduction of pTShygMRS35RRubipt.
Fig. 14 shows the results of PCR analysis on part of a rooting individual, obtained in Example 1-V1.
Fig. 15 shows a construction scheme of pTL7MRS36RRubipt, a vector for introduction of a recombinase gene.
Fig. 16 shows a positional relationship between the primer used in Example 2 of the present invention and a chromosome which may exist in plant cells after introduction of pTL7MRS36RRubipt.
Fig. 17 shows the results of PCR analysis with the rooting individual, obtained in Example 2-III.
Fig. 18 shows a construction scheme of pTL735R and pTL7rubipt35R, vectors for introduction of recombinant enzyme gene.
Fig. 19 shows positional relationships between the primers used in Examples 3 and 4 of the present invention and chromosomes which may exist in plant cells after introduction of pTL735R or pTL7rubipt35R.
Fig. 20 shows the results of PCR analysis with parts of rooting individuals, obtained in Examples 3-III and 4-III.

### BEST MODE FOR CARRYING OUT THE INVENTION

First, details of the first embodiment of the present invention is described.

In the first embodiment of the present invention, first of all, a vector comprising two site-specific recombinase recognition sequences (hereinafter, also simply referred to as a recognition sequence), oriented in the opposite direction, and a site-specific recombinase gene (hereinafter, also simply referred to as a recombinase gene) encoding a site-specific recombinase (hereinafter, also simply referred to as a recombinase) which recognizes the recognition sequence, positioned inside or outside of the region between the two recognition sequences, is introduced into a plant cell.

The recognition sequence of a site-specific recombinase is a DNA factor having a predetermined nucleotide sequence; in a site-specific recombination system, the site-specific recombinase recognizes the recognition sequence to exert various functions. For example, when there are two regions between two recognition sequences of the same nucleotide sequence on a DNA molecule (X-X'), positioned in the opposite direction (i.e., recognition sequences oriented in the opposite direction, in the present invention), recombination occurs in the regions between these X-X' regions through the function of the recombinase regardless of whether these two regions exist on the same DNA molecule or on different DNA molecules separately. In the present invention, the function is used.

As known site-specific recombination systems, the combinations of recognition sequences and recombinases which recognize them to exert functions include the Cre/lox system, R/RS system, FLP/FRT system, cer system and fim system isolated from microorganisms such as phage, bacteria (*E. coli, etc*.) and yeast (for review of article, N. L. Craig, Annu. Rev. Genet., 22: 17 (1988)). No site-specific recombination system in higher organisms is known yet, however, it has been demonstrated that the recognition sequences and recombinases of the site-specific recombination systems, isolated from the above-described microorganisms, behave in the same manner as in the original organisms, even if introduced into organism species other than those from which the systems derived, i.e., plants and animals.

The recognition sequence and recombinase gene used in the present invention can be selected arbitrarily from combinations of these recognition sequences and genes encoding a recombinase which recognizes them to function. In particular, the Cre/lox system and R/RS system are widely used in animals, plants, *etc.,* showing high recombination efficiency, and therefore are preferable as combinations of recognition sequences and recombinase genes used in the present invention. Mutated types (i.e., wild type recognition sequences with artificial or natural partial modifications) can also be used as recognition sequences. However, the mutated recognition sequences in this case should be recognizable by the recombinase encoded by the recombinase gene which coexists in the above-described vector.

In addition, the present invention allows more efficient isolation of cells in which chromosomes are disappeared with use of a negative selectable marker gene. Specifically, the negative selectable marker gene is used by being positioned inside or outside of the region between the recognition sequences of the above-described vector. Here, the positional relationship with the recombinase gene makes no difference. Specifically, the negative selectable marker gene can be positioned inside or outside of the recognition sequence together with recombinase gene, or the recombinase gene can be positioned inside and the negative selectable marker gene can be positioned outside, or vice versa.

Any genes whose expression inhibits normal growth and differentiation of host cells can be used as a negative selectable marker gene. Such genes include lethal induction genes and morphological abnormality induction genes.

The lethal induction genes indicate overall genes whose expression impairs the functions of host cells and leads to death; for example, genes encoding RNAse, DAM methylase, cytosine deaminase (codA), diphteria toxin, Bax and so forth are widely known. The morphological abnormality induction genes indicate overall genes whose expression disrupts the directions of host cell growth and differentiation and causes morphological differentiation out of the ordinary. Plant hormone-associated genes such as plant hormone synthesis genes and plant hormone signal transduction genes which cause growth retardation of a host plant, loss of apical dominance, change of pigment, crown gall, callus induction, hairy root, rippling leaves and so forth can be used as the morphological abnormality induction genes.

The plant hormone synthesis genes are genes which encode proteins involved in the plant hormone synthesis [e.g., *ipt* (isopentenyltransferase) gene of plant pathogens such as *Agrobacterium* (A. C. Smigocki, L. D. Owens, Proc. Natl. Acad. Sci. USA, 85: 5131 (1988)), *iaaM* (tryptophan monooxygenase) gene (H. J. Klee et al., GENES & DEVELOPMENT, 1 : 86 (1987)), gene 5 gene (H. Kerber et al., EMBO Journal, 10 : 3983 (1991)), gene *6b* gene (P. J. J. Hooyaas et al., Plant Mol. Biol., 11: 791 (1988)), *rol* gene groups such as *rolA to rolD* (F. F. White et al., J. Bacteriol., 164 : 33 (1985)), *iaaL* (indoleacetic acid-lysine synthetase) gene of *Pseudomonas syringae* subsp. *savastanoi* (A. Spena et al., Mol Gen. Genet., 227: 205 (1991)), and in addition, homeobox genes, phytochrome genes and so forth of various plants].

Plant hormone signal transduction genes are genes which encode proteins of sensors which recognize the existence of plant hormones such as gibberellin, ethylene, auxin and cytokinin or proteins involved in a series of signal transduction pathways from the sensors, [e.g., *ETR1* gene (ethylene receptor gene) (C. Chang et al., Science, 262: 539 (1993)), *CKI1* gene (cytokinin receptor gene) (T. Kakimoto, Science, 274: 982 (1996) and variants thereof (e.g., *CKI2* gene), *GCR1* gene (S. Plakidou-Dymock et al., Current Biology, 8: 315 (1988)), *IBC6* gene and *IBC7* gene (I. Brandstatter, J. J. Kieber, The Plant Cell, 10: 1009 (1988))].

Among these morphological abnormality induction genes, the *ipt* gene which causes loss of apical dominance and *rol* genes which cause formation of hairy root, growth retardation of a plant reproduced from hairy root, rippling leaves and so forth induce characteristic morphological abnormalities to inhibit redifferentiation of a plant individual and are therefore particularly preferable as the morphological abnormality induction genes used in the present invention.

The method for introducing the vector of the present invention into a plant cell includes direct and indirect introducing methods; physical or chemical methods such as microinjection, electroporation, polyethylene glycol method, fusion method, and high-speed ballistic penetration method can be used as direct introducing methods, and techniques with viruses and bacteria which infect plants can be used as indirect introducing methods (I. Potrykus, Annu. Rev. Plant Physiol. Plant Mol. Biol., 42: 205 (1991)). Among the viruses and bacteria used for the indirect introducing methods, viruses include cauliflower mosaic virus, geminivirus, tobacco mosaic virus and brome mosaic virus, and representative bacteria include *Agrobacterium tumefaciens* (hereinafter, abbreviated as *A. tumefaciens*) and *Agrobacterium rhizogenes.*

The vector-introduced plant cells can be cultured and grown under heretofore known culturing and environmental conditions most suitable for plant cells. Cells in which one or more chromosomes are disappeared are produced among grown cells, and DNA analysis on the cells obtained from cultured tissue after growth allows selection of cells with target chromosomes disappeared or tissues containing the cells. Here, the heretofore known techniques such as PCR and Southern blotting can be used as techniques for the DNA analysis. When a vector comprising a negative selectable marker gene is used as the vector, cells in which chromosomes are disappeared can be selectively obtained by culturing vector-introduced plant cells under conditions where the negative selectable marker gene inhibits the normal growth and differentiation of cells having this on chromosomes (in the present invention, these conditions are referred to as conditions where a negative selectable marker gene functions).

Next, the second embodiment of the present invention is described in detail.

In the second embodiment of the present invention, a vector comprising one site-specific recombinase recognition sequence comprising a point-symmetric nucleotide sequence, and a site-specific recombinase gene encoding a site-specific recombinase which recognizes the site-specific recombinase recognition sequence is used as the vector to be introduced into a plant cell.

Here, the site-specific recombinase recognition sequence comprising a point-symmetric nucleotide sequence is a DNA factor with a so-called palindromic sequence in which a predetermined nucleotide sequence is arranged in a point-symmetric manner. This recognition sequence comprising a point-symmetric nucleotide sequence is the same as the recognition sequence used in the first embodiment of the present invention in that it functions in combination with the recombinase which recognizes this recognition sequence to function, but is different in that two copies of this recognition sequence simply exist on a DNA molecule and recombination of the DNA molecule occurs via the function of the recombinase regardless of whether they exist on the same DNA molecule or different DNA molecules. Recognition sequence LoxPsym which functions in combination with the recombinase of the Cre/lox system is known as the recognition sequence comprising a point-symmetric nucleotide sequence (Ronald H. Hoess et al., Nucleic Acid Res., 11: 14(5): 2287-2300 (1986)).

Also, in the present invention, a negative selectable marker gene is used in the same manner as in the first embodiment of the present invention to further improve the efficiency of obtaining cells in which chromosomes are disappeared. For this purpose, this negative selectable marker gene is positioned in the above-described vector together with the recognition sequence comprising the above point-symmetric nucleotide sequence and recombinase gene. Here, the positional relationship with the recognition sequence or recombinase gene makes no difference. As the negative selectable marker gene, the negative selectable marker gene in the first embodiment of the present invention, described above, can also be used in the present invention in the same manner.

As the items other than those specifically described, the present invention can be conducted in the same manner as in the first embodiment of the present invention. Specifically, the vector of the present invention can be introduced into a plant cell in the same manner as in the first embodiment of the present invention, and the plant cells to which the vector of the present invention was introduced are cultured and grown to produce cells in which one or more chromosomes are disappeared, and these cells can be selected in the same manner as in the first embodiment of the present invention.

Furthermore, the third embodiment of the present invention is described in detail.

In the third embodiment of the present invention, first of all, a vector comprising two site-specific recombinase recognition sequences, oriented in the opposite direction, or one site-specific recombinase recognition sequence comprising a point-symmetric nucleotide sequence, is used as a vector to be introduced into a plant cell (hereinafter, this vector is also referred to as a vector for introduction of a recognition sequence). Unlike in the vectors in the first and second embodiments of the present invention, a recombinase gene encoding a recombinase which recognizes these recognition sequences is not positioned in this vector.

On the other hand, a negative selectable marker gene is also positioned in this vector together with these recognition sequences in the same manner as in the vectors in the first and second embodiments of the present invention to further improve the efficiency of obtaining cells in which chromosomes are disappeared. In order to position this negative selectable marker gene together with two recognition sequences oriented in the opposite direction, this negative selectable marker gene can be positioned inside or outside of the region between the recognition sequences. In order to position this negative selectable marker gene together with one recognition sequence comprising a point-symmetric nucleotide sequence, this negative selectable marker gene can be positioned regardless of its positional relationship.

In the present invention, after introduction of the above-described vector into a plant cell, the recombinase which recognizes the recognition sequence positioned in this vector is allowed to act transiently in the cells during at least growth of the cells.

In order to allow the recombinase to act at least transiently in growing plant cells, for example, the recombinase itself or a recombinase gene encoding this recombinase is introduced into the growing plant cells, and/or after introduction into the plant cells, the plant cells are grown.

As the introduction of the recombinase gene, the recombinase can be allowed to act transiently in the cells only by positioning this recombinase gene in an appropriate vector to be introduced into a plant cell (hereinafter, the vector used for the introduction of the recombinase gene into a plant cell after introduction of the recognition sequence is also referred to as a vector for introduction of a recombinase gene). This is because the recombinase gene introduced into a plant cell is expressed without being incorporated into the chromosomes, and the free recombinase gene disappears after producing the recombinase to some extent and stops production of the recombinase in the cells, to thereby allow this recombinase to act transiently.

However, in order to ensure such transient action of the recombinase, the recombinase gene between two recognition sequences comprising the same nucleotide sequence, oriented in the same direction, and recognized by the recombinase that this recombinase gene encoded (i.e., recognition sequence oriented in the same direction, in the present invention), is positioned in a vector to be introduced into the plant cells. The recombinase gene, thus introduced into the plant cells, is incorporated and expressed in a state of being flanked by the above two recognition sequences even if incorporated into the chromosomes of the plant cells; thus, after producing the recombinase to some extent in the cells, gene is removed and freed from the chromosomes, and then disappeared by the function of recombinase produced by the gene itself.

When the recombinase is allowed to act transiently in the cells by introducing the recombinase gene into the plant cells, cells in which the recombinase has been allowed to act transiently can reliably be obtained from cells, to which the recombinase gene is introduced, by introducing a negative selectable marker gene into the plant cells together with the recombinase gene. As the negative selectable marker gene, the negative selectable marker gene used in the above-described first and second embodiments of the present invention can be used in the same manner also in the present invention.

In the above case, a negative selectable marker gene is introduced into a plant cell, together with a recombinase gene, only by positioning this recombinase gene and negative selectable marker gene in a vector and introducing the vector into the plant cells. In order to position a recombinase gene between two recognition sequences oriented in the same direction in a vector, a negative selectable marker gene is positioned, together with the recombinase gene, between two recognition sequences oriented in the same direction in the vector.

When the recombinase gene is introduced into a plant cell to which the recombinant enzyme has been allowed to act transiently in the cells, it is preferred to introduce an additional selectable marker gene into the plant cells together with the recombinase gene. Specifically, in the same manner as in the negative selectable marker gene described above, a selectable marker gene is positioned in a vector together with the recombinase gene, or with the recombinase gene and negative selectable marker gene, and this vector is introduced into the plant cells. In this case, positioning, type, the number and so forth of the selectable marker gene are not particularly limited. For example, when the recombinase gene is positioned between two recognition sequences oriented in the same direction, the selectable marker gene can be positioned inside or outside of the region between the recognition sequences, or can be positioned with the region between the recognition sequences existing as an obstacle between the promoter region and structural gene region of the selectable marker gene so that disappearance of the recombinase gene results in the expression of the selectable marker gene.

One or more heretofore known selectable marker genes, including the neomycin phosphotransferase (NPTII) gene which confers kanamycin resistance to plants, antibiotic resistance genes such as the hygromycin phosphotransferase gene which confers hygromycin resistance to plants and herbicide tolerance genes such as the phosphinothricin acetyltransferase (bar) gene which confers bialaphos resistance, can be used as selectable marker genes. Thus, introducing a selectable marker gene into a plant cell together with a recombinase gene allows selective growth of cells to which a recombinase gene was introduced.

The plant cells, to which a recombinase or recombinase gene encoding a recombinase is introduced, and in which this recombinase has been allowed to act transiently during growth, can be cultured and grown under the heretofore known culturing and environmental conditions most suitable for plant cells. Cells in which one or more chromosomes are disappeared are produced among grown cells, and DNA analysis on the cells obtained from cultured tissue after growth allows selection of cells with target chromosomes disappeared or tissues containing the cells. When a vector comprising a negative selectable marker gene is used as the vector for introduction of a recognition sequence or recombinase gene, cells in which chromosomes are disappeared and/or cells in which a recombinase has been allowed to act transiently can be selectively obtained by culturing the plant cells, into which the vector for introduction of the recombinase gene is introduced, under conditions where the negative selectable marker gene functions.

As items other than those specifically described, the present invention can be conducted in the same manner as in the first or second embodiment of the present invention. Specifically, in the present invention, the recognition sequence and recombinase gene used in the vector in the first embodiment of the present invention can be used as two recognition sequences oriented in the same or opposite direction and recombinase gene encoding a recombinase which recognizes the sequences to exert function; the recognition sequence and recombinase gene used in the vector in the second embodiment of the present invention can be used as a recognition sequence comprising a point-symmetric nucleotide sequence and recombinase gene encoding a recombinase which recognizes this to exert function. The vector for introduction of a recognition sequence and the vector for introduction of a recombinase gene can be introduced into a plant cell in the same manner as in the first embodiment of the present invention, and the plant cells to which the vector for introduction of a recognition sequence can be cultured in the same manner as in the first embodiment of the present invention.

### <Effects>

In the cells for which two recognition sequences oriented in the opposite direction are introduced into chromosomes, the chromosomes are replicated and duplicated as somatic cell division (mitosis) starts. At this time, if the recombinase functions, DNA recombination may occur in the regions between two recognition sequences, which exist on each of these two chromosomes produced by duplication.

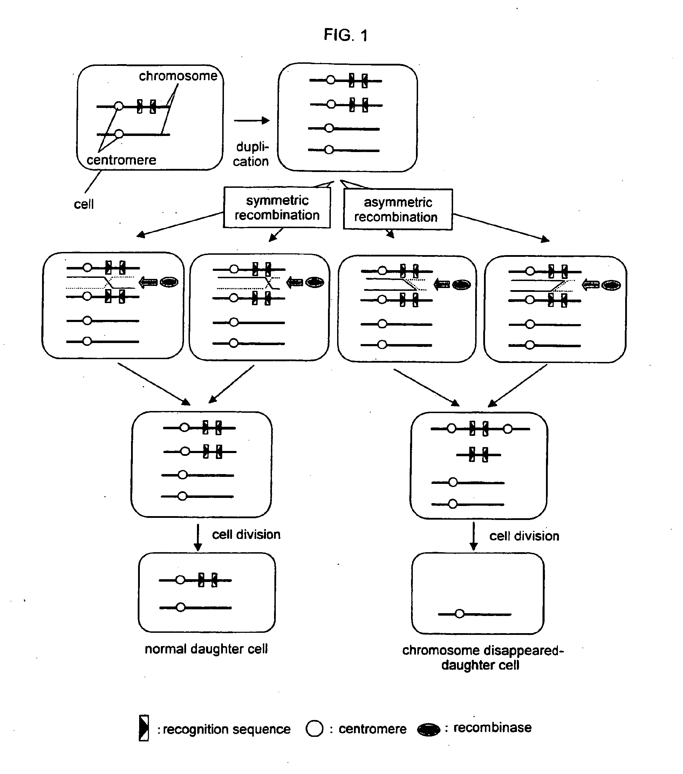

When this recombination occurs symmetrically, there is no substantial change in both chromosomes after recombination. However, when this recombination occurs asymmetrically, one chromosome having two centromeres in a point-symmetric position (dicentric chromosome) and one chromosome without centromere (acentric chromosome) are produced after recombination. In normal somatic cell division, the duplicated chromosomes move to both poles of the spindle fibers which are subsequently produced, and are divided while moving along the spindle fibers to be chromosomes of newly-produced daughter cells, however, the dicentric chromosome and acentric chromosome cannot move along the spindle fibers and therefore cannot be divided into daughter cells. Thus, both daughter cells which are produced after somatic cell division at this time lose the corresponding chromosomes (Fig. 1).

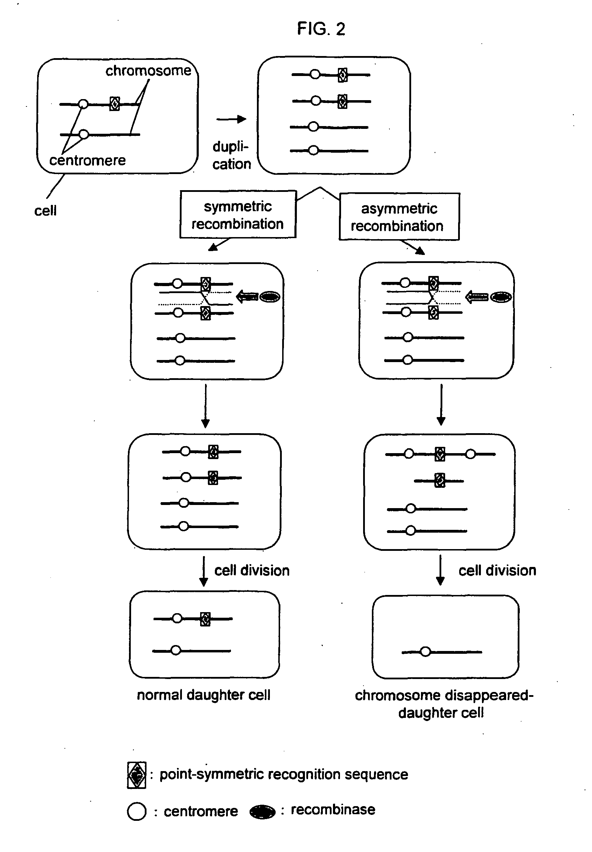

The same phenomenon also occurs in cells to which one recognition sequence comprising a point-symmetric nucleotide sequence is introduced into chromosomes. This is because when a recognition sequence comprising a point-symmetric nucleotide sequence is introduced into chromosomes, DNA recombination occurs between the recognition sequences which exist on each of the two chromosomes produced by duplication, and also in this case, as a result of asymmetric recombination, the dicentric chromosome and acentric chromosome are produced all the same (Fig. 2).

As a result of keen examination, the inventors made the above finding and achieved the first, second and third embodiments of the present invention.

Specifically, in the first embodiment of the present invention, a vector comprising two recognition sequences oriented in the opposite direction and a recombinase gene encoding a recombinase which recognizes the recognition sequence, positioned inside or outside of the region between the two recognition sequences, is introduced into a plant cell, and these cells are cultured and grown.

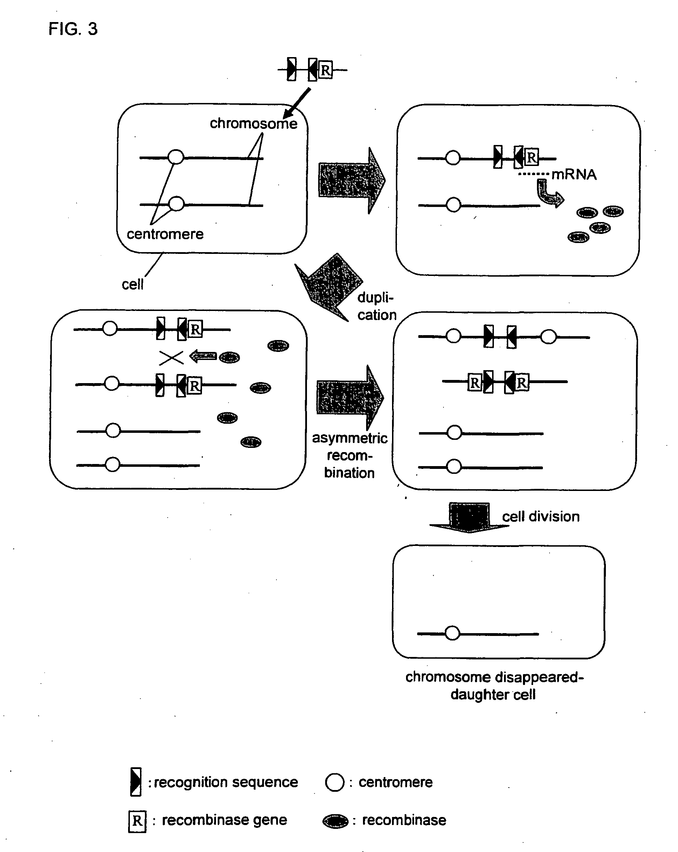

If two recognition sequences oriented in the opposite direction and a recombinase gene encoding a recombinase which recognizes the recognition sequence are introduced into the chromosomes of these plant cells by introduction of the above-described vectors into a plant cell, two chromosomes with the same structure introduced are produced after somatic cell division starts during the growth of these plant cells. At this time, the functions of the recombinase produced by the expression of the recombinase gene on the chromosomes cause a DNA recombination in the regions between the two recognition sequences which exist on these two chromosomes, and as a result, a dicentric chromosome and acentric chromosome are produced to generate daughter cells in which chromosomes are disappeared (Fig. 3).

In the second embodiment of the present invention, a vector comprising one recognition sequence comprising a point-symmetric nucleotide sequence and a recombinase gene encoding a recombinase which recognizes the recognition sequence is introduced into a plant cell, and these cells are cultured and grown.

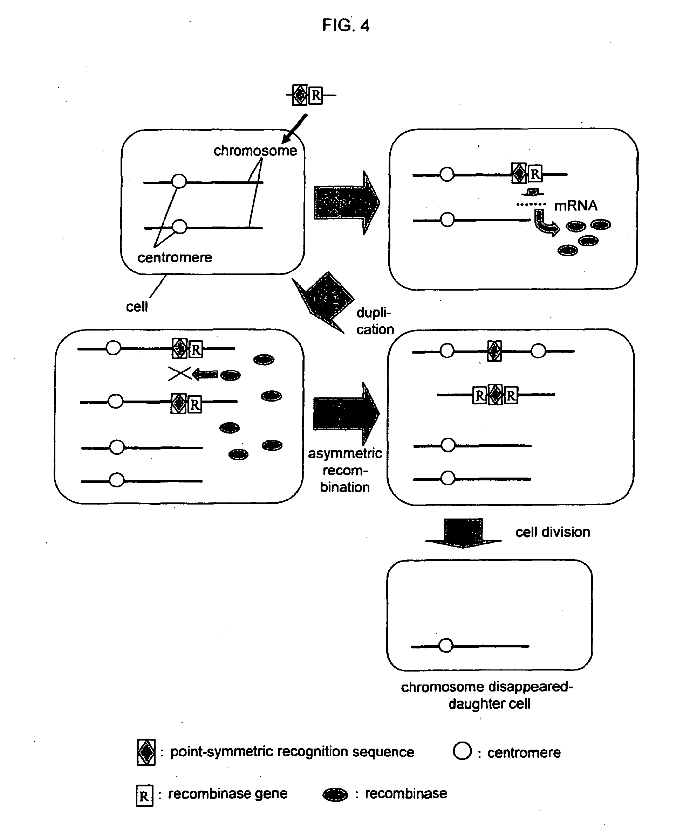

Also, in this case, daughter cells in which chromosomes are disappeared are produced in the same manner as in the first embodiment of the present invention. One different point from the case in the first embodiment of the present invention is that DNA recombination occurs between two recognition sequences which exist on each of the duplicated two chromosomes (Fig. 4).

In the third embodiment of the present invention, after a vector comprising two recognition sequences oriented in the opposite direction or one recognition sequence comprising a point-symmetric nucleotide sequence is introduced into a plant cell, the recombinase which recognizes the above-described recognition sequence functions is allowed to act during the growth of the cells.

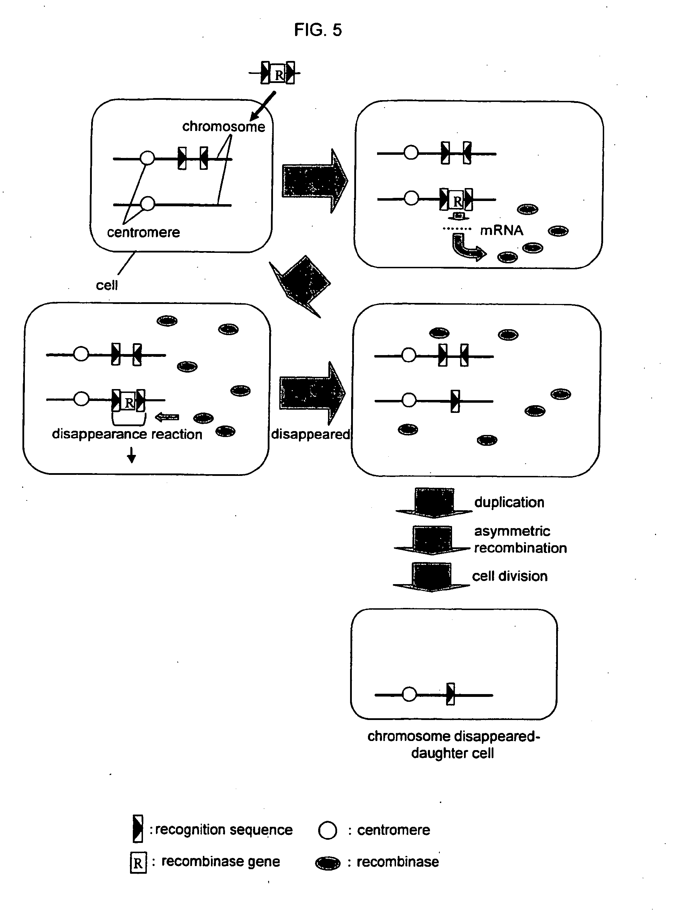

For example, the following description is given for the case where a vector for introduction of a recognition sequence, which carries two recognition sequences oriented in the opposite direction, is introduced into a plant cell, and subsequently a vector for introduction of a recombinase gene, having a recombinase gene between by recognition sequences oriented in the same direction, is introduced into the plant cells to allow the recombinase to act transiently during the growth of the cells (Fig. 5): in the third embodiment of the present invention, introduction of a vector for introduction of a recognition sequence produces chromosomes into which two recognition sequences oriented in the opposite direction are introduced, and subsequently introduction of a vector for introduction of a recombinase gene into these cells causes the expression of the recombinase gene that this vector carries and produces a recombinase. In this case, if the cells are cultured and grown, the functions of the recombinase cause DNA recombination in the regions between the two recognition sequences which exist on the two chromosomes which carry two recognition sequences oriented in the opposite direction, produced by duplication by the start of somatic cell division, and as a result, a dicentric chromosome and acentric chromosome are produced to produce daughter cells in which chromosomes are disappeared.

The recombinase gene, thus positioned in the vector for introduction of a recombinase gene and introduced into a plant cell, is incorporated and expressed in such a state it exists between the two recognition sequences oriented in the same direction even if incorporated into chromosomes of the plant cells; thus, after the recombinase is produced to some extent, the gene is disappeared and freed from the introduced chromosomes, and then disappears by the function of the recombinase produced by the gene itself. Thus, the recombinase functions transiently. With the vector of the third embodiment of the present invention, the transient function of a recombinase is ensured through such mechanisms and thus negative effects such as DNA instability due to constant expression of a recombinase gene in cells with target chromosomes disappeared can be prevented.

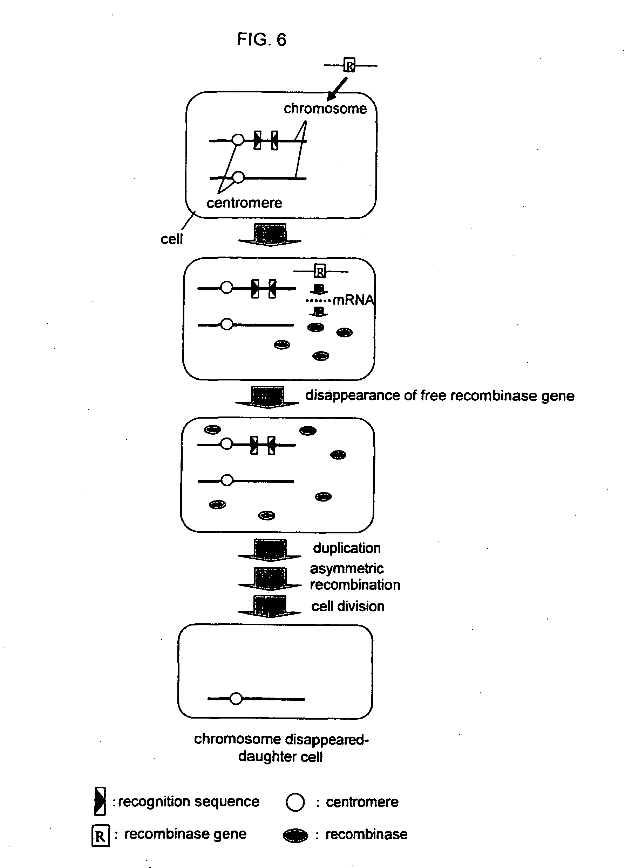

However, even a vector without a recognition sequence, having only this recombinase gene, can be used as a vector for introduction of a recombinase gene. As described above, the recombinase gene introduced into a plant cell can be expressed without being incorporated into chromosomes to allow transient function of a recombinase. Therefore, even a vector without a recognition sequence, having a recombinase gene, allows transient function of a recombinant enzyme in plant cells, into which the vector is introduced, by the functions of the free recombinase gene. When a recombinase is allowed to act transiently through such mechanism, the target chromosome is disappeared, and cells leaving no trace of the vector for introduction of the recombinase gene, as well as no trace of the vector for introduction of the recognition sequence (i.e., cells leaving no trace of genetic manipulation) can be obtained (Fig. 6).

When a negative selectable marker gene is positioned in the vector used in the above-described first or second embodiment of the present invention or the vector for introduction of a recognition sequence, used in the third embodiment of the present invention, cells in which chromosomes are disappeared can be obtained more efficiently.

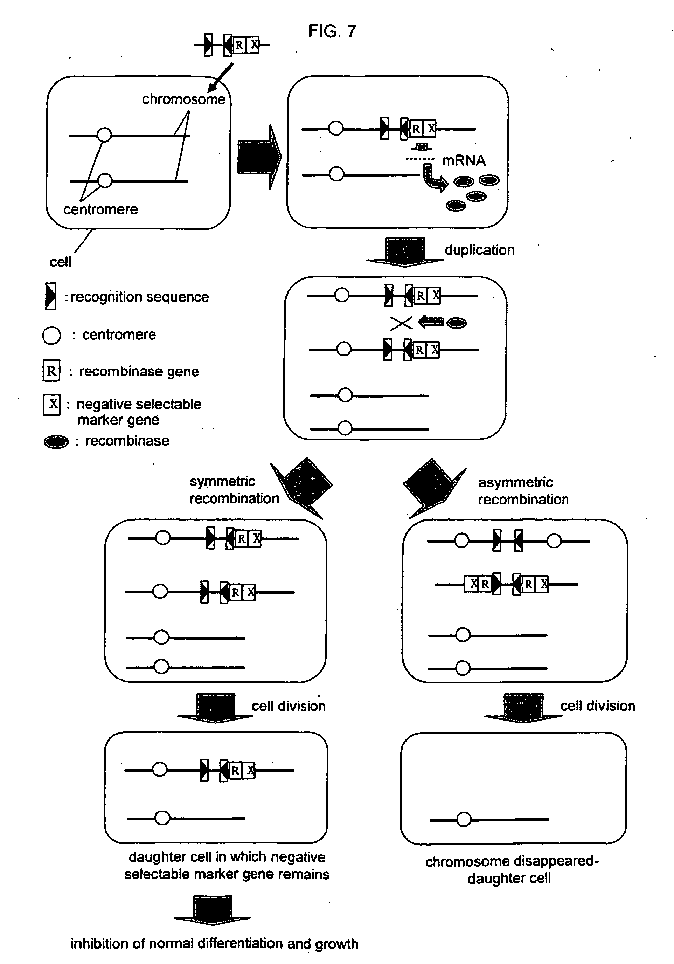

For example, when a vector comprising a negative selectable marker gene, outside of two recognition sequences oriented in the opposite direction together with a recombinase gene, in the first embodiment of the present invention is introduced into a plant cell (Fig. 7), a negative selectable marker gene remains in the daughter cells which are obtained after symmetric recombination in the regions between the two recognition sequences on the two chromosomes produced by duplication, having two recognition sequences oriented in the opposite direction, and thus normal growth and differentiation are inhibited under conditions where the negative selectable marker gene functions. Therefore, as a result of asymmetric recombination, only daughter cells in which chromosomes are disappeared can be selectively obtained.

In the third embodiment of the present invention, the above-described negative selectable marker gene can be positioned together with a recombinase gene in the vector for introduction of a recombinase gene.

For example, when a vector comprising a recombinase gene without a recognition sequence is used as a vector for introduction of a recombinase gene, and the recombinase gene positioned in this vector is introduced into a chromosome other than those to which the recognition sequence was introduced by the vector for introduction of a recognition sequence, the recombinase gene remains on the chromosome to which the gene was introduced, even after the target chromosome is disappeared, and is expressed constantly to produce a recombinase with resultant negative effects such as DNA instability.

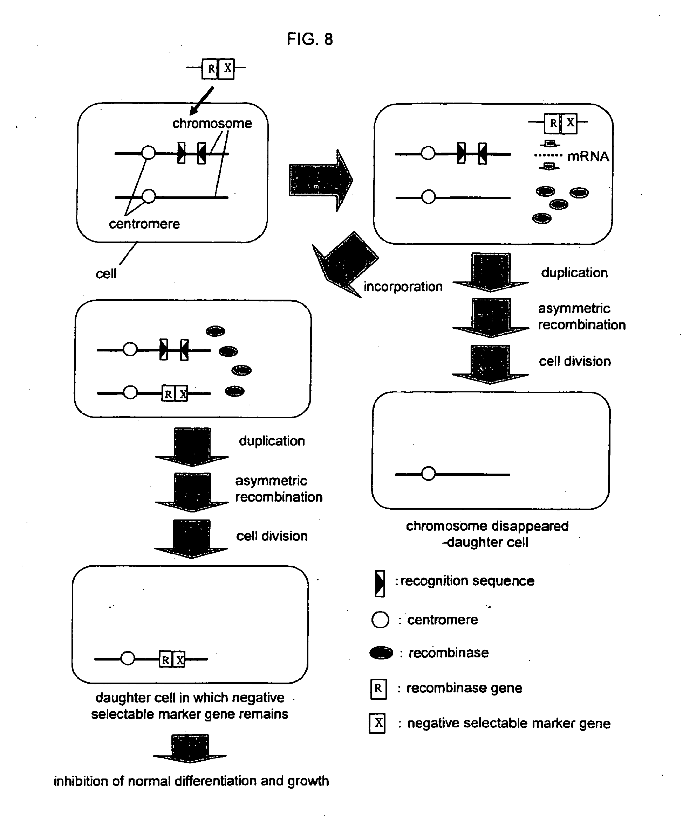

However, in this case, a vector comprising a negative selectable marker gene together with a recombinase gene is used as a vector for introduction of a recombinase gene, the negative selectable marker gene is also introduced to and remains on the chromosome even if a recombinase gene is introduced to and remains on the chromosome other than those to which the recognition sequence was introduced by a vector for introduction of a recognition sequence; thus, normal growth and differentiation are inhibited by culturing under conditions where the negative selectable marker gene functions. Said cells are thus disappeared, and cells to which the recombinase has been allowed to act transiently can reliably be obtained (Fig. 8).

When a recombinase gene is positioned between two recognition sequences oriented in the same direction in a vector for introduction of a recombinase gene, cells in which the recombinase has been allowed to act transiently can be obtained more reliably by positioning a negative selectable marker gene between the two recognition sequences together with the recombinase gene.

When a vector for introduction of a recombinase gene in which a recombinase gene is positioned in such a state that it exists between two recognition sequences oriented in the same direction is introduced into a plant cell, the recombinase gene usually produces a recombinase to some extent even if incorporated into the chromosomes of plant cells, and is disappeared and freed from the introduced chromosomes and subsequently disappears (Fig. 5), but in some cases, this recombinase gene continues to be expressed without being disappeared from the introduced chromosomes and causes the above-described negative effects.

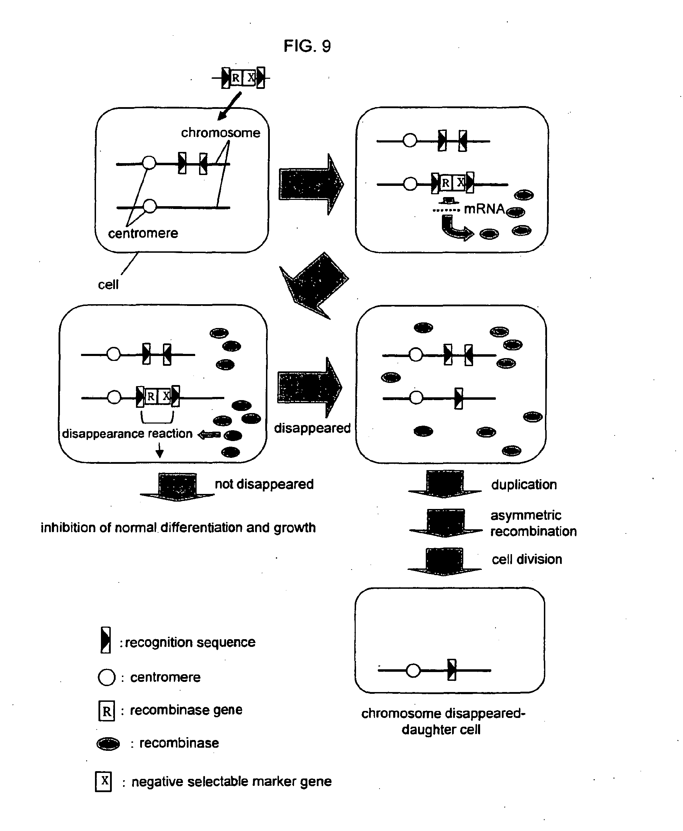

However, also in this case, when a negative selectable marker gene is positioned in a vector for introduction of a recombinase gene as described above, this negative selectable marker gene behaves in the same manner as the recombinase gene for site-specific recombination; thus, the negative selectable marker gene also continues to exist on the chromosomes of cells when the recombinase gene continues to be expressed without being disappeared after introduction into the chromosomes of plant cells, and culturing under conditions where this negative selectable marker gene functions inhibits normal differentiation and growth of cells and eliminates the cells (Fig. 9).

Hereinafter, the details of the present invention is described according to examples. However, the present invention is not limited to the examples described below. In the following examples, if not otherwise specified, further details of the experimental procedures followed Molecular Cloning (Sambrook et al. (1989)) or the manufacturer's instructions.

### Example 1

### I. Construction of a vector for introduction of recognition sequence (pTSspsRScodN)

A plasmid, pCRScodN, having the NPTII structural gene linked with the promoter of the nopaline synthase gene (Nos-P) and the polyadenylation signal of the nopaline synthase gene (hereinafter, all polyadenylation signals are those derived from nopaline synthase) and codA structural gene linked with the 35S promoter (35S-P) and polyadenylation signal, oriented in the opposite direction, between recognition sequence RS derived from site-specific recombination system R/RS, was constructed. The codA structural gene was obtained by PCR method using genomic DNA of *E. coli* (DH5α, purchased from Toyobo Co., Ltd.) as a template and primer α represented by SEQ ID NO:1 (5'-gctctagagc atgtggaggc taacagtg-3') and primer β represented by SEQ ID NO:2 (5'-gcgagctctc agtgctctac gtaggccg-3').

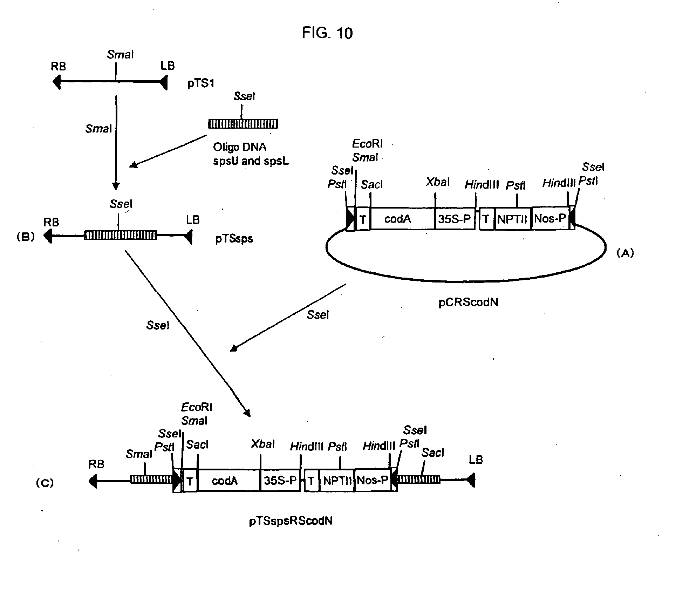

The structure of pCRScodN is shown in Fig. 10A. In the figure, the black triangle in the box indicates the directions of RS and its sequence, and T indicates the polyadenylation signal (same below).

A plasmid, pTSsps, was constructed by inserting a DNA fragment obtained by annealing oligonucleotide, spsU, represented by SEQ ID NO:3 (5'-tgcagaataa ataaacgcca tggcacccgg gaaagaaata aaatgaaacg caaacacatg acctgcaggc acataagatg atacgcaagc gccgagctct actacgcaat ggcagacgca aaagcaggct acgcatgca) and oligonucleotide, spsL, represented by SEQ ID NO:4 (5'-tgcatgcgta gcctgctttt gcgtctgcca ttgcgtagta gagctcggcg cttgcgtatc atcttatgtg cctgcaggtc atgtgtttgc gtttcatttt atttctttcc cgggtgccat ggcgtttatt tattctgca-3') into the restriction enzyme *Sma*I site of plasmid pTS1 (W02004/87910) (Fig. 10b), and the two RSs oriented in the opposite direction and the region therebetween, excised from the above-described pCRScodN with the restriction enzyme *Sse*I, was inserted into the restriction enzyme *Sse*838701 site (hereinafter, abbreviated as *Sse*I) of this pTSsps to construct a vector plasmid, pTSspsRScodN, for the introduction of a recognition sequence into host DNA [Fig. 10C: domestically deposited to the International Patent Organism Depositary, National Institute of Advanced Industrial Science and Technology (Address: Central 6, 1-1-1 Higashi, Tsukuba, Ibaraki 305-8566 Japan) on December 3, 2004 (Deposit No. FERM P-20312) or internationally deposited on December 8, 2005].

When this vector is introduced into a plant cell, the region between the right border (RB) and the left border (LB) derived from the T-DNA, indicated by the black triangles in the figure, is introduced into chromosomes. The codA gene is a gene encoding an enzyme which converts 5-fluorocytosine (5-FC) into cytotoxic 5-fluorouracil; thus, when plant cells to which codA gene is introduced into the chromosomes and expressed are cultured in a 5-FC supplemented medium, the cells are killed; thus, in this example, as described in the following II in detail, this gene is used as a negative selectable marker gene to selectively obtain cells in which chromosomes are disappeared. The NPTII gene functions as a selectable marker gene to select plant cells to which the above-described RB-LB region is introduced into chromosomes by this vector.

### II. Construction of a vector for introduction of recombinase gene (pTShygMRS35RRubipt)

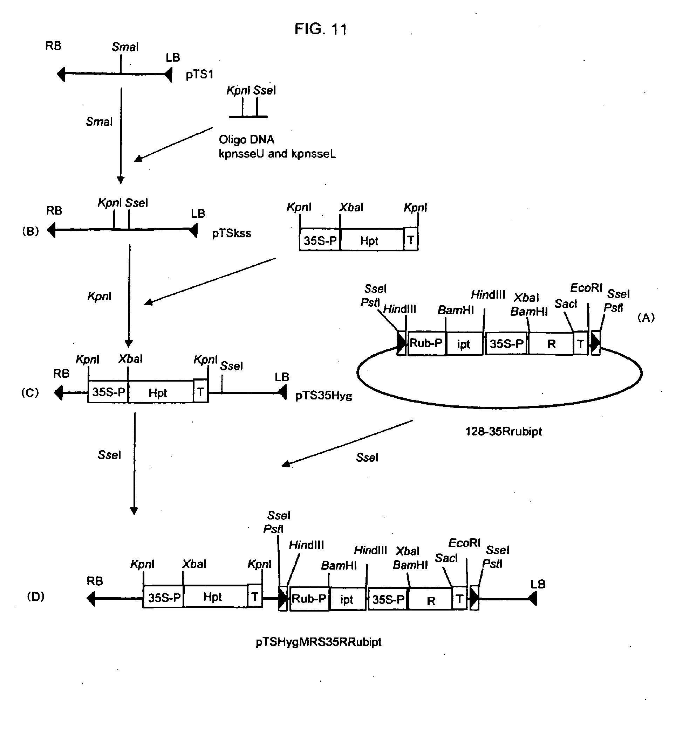

A plasmid, 128-35Rrubipt, having the *ipt* gene linked with the Rubisco promoter (Rub-P) and site-specific recombinase (R) structural gene linked with the 35S-P and polyadenylation signal, between RSs oriented in the same direction, was constructed (Fig. 11A).

A plasmid, pTSkss, was constructed by inserting a DNA fragment obtained by annealing oligonucleotide, kpnsseU, represented by SEQ ID NO:5 (5'-ctgcacgcta cctgcagg-3') and oligonucleotide, kpnsseL, represented by SEQ ID NO:6 (5'-cctgcaggta gcgtgcag-3') into the restriction enzyme *Sma*I site of plasmid pTS1 (WO2004/87910) (Fig. 11B), and a structural gene of the hygromycin (*Hpt*) resistant gene linked with the 35S-P and polyadenylation signal was inserted into the restriction enzyme KpnI site of this pTSkss to construct a plasmid, pTS36Hyg (Fig. 11C).

Subsequently, the RSs and the region therebetween are excised from the 128-35Rrubipt with restriction enzyme *Sse*I, was linked with the restriction enzyme *Sse*I site of the above-described pTS35Hyg to construct a vector plasmid, pTShygMRS35RRubipt, for the introduction of the recombinase gene [Fig. 11D : domestically deposited to the International Patent Organism Depositary, National Institute of Advanced Industrial Science and Technology (Address: Central 6, 1-1-1 Higashi, Tsukuba, Ibaraki 305-8566 Japan) on December 3, 2004 (Deposit No. FERM P-20313) or internationally deposited on December 8, 2005]. When this vector is introduced into a plant cell, the region between the RB and the LB derived from the T-DNA, indicated by the black triangles in the figure, is introduced into chromosomes.

When the vector constructed here for the introduction of the recombinase gene, pTShygMRS35RRubipt, is introduced into a plant cell to which the above-described vector for introduction of the recognition sequence, pTSspsRScodN, was introduced, the R gene positioned in this pTShygMRS35RRubipt is expressed to produce the recombinase. In this case, when the cells are cultured and grown, the two chromosomes produced by duplication at the start of somatic cell division, having two RSs derived from pTSspsRScodN, oriented in the opposite direction, cause DNA recombination in the regions between the above-described two RSs by the function of the recombinase.

Among the cells in which such DNA recombination has been caused, a dicentric chromosome and acentric chromosome are produced in cells in which asymmetric recombination has been caused; as a result, daughter cells are produced, in which the structures derived from the above-described pTSspsRScodN (i.e., two RSs oriented in the opposite direction and chromosomes having the codA gene and others which exist in the region between these two RSs) are lost. However, in cells in which symmetric recombination has been caused, there is no substantial change in the chromosome after recombination. Thus, a chromosome with the structure derived from pTSspsRScodN is maintained. Therefore, when pTShygMRS35RRubipt is introduced into a plant cell to which pTSspsRScodN has been introduced and the cells are cultured in the presence of 5-FC, cells in which symmetric recombination has been caused are killed by the function of the codA gene which remains in the chromosome, and only cells in which chromosomes are disappeared by asymmetric recombination can be selected.

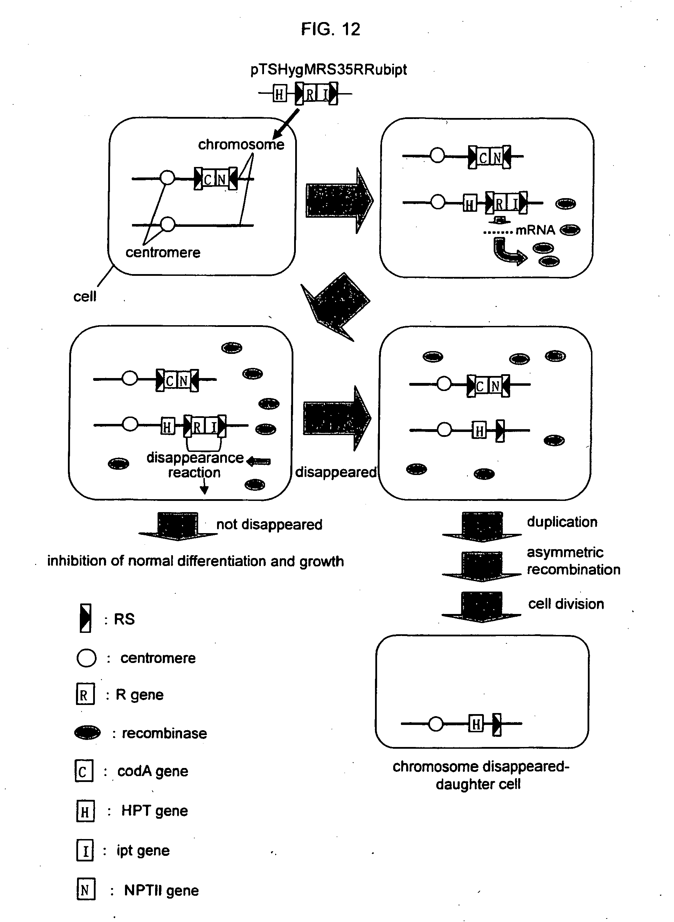

The R gene positioned in pTShygMRS35RRubipt, thus introduced into a plant cell, is incorporated and expressed in a state that it exists between the RSs oriented in the same direction even if incorporated into chromosomes of plant cells; thus, after a recombinase is produced to some extent, the gene is disappeared and freed together with the *ipt* gene from the chromosome, and then disappears by the function of the recombinase produced by the gene itself Thus, the recombinase is allowed to act transiently (Fig. 12).

When the R gene is not disappeared from chromosomes through the above-described mechanism in the pTShygMRS35RRubipt, the *ipt* gene functions as a negative selectable marker gene to eliminate relevant cells, and the *Hpt* gene functions as a selectable marker gene to select plant cells having chromosomes into which the above-described RB-LB region is introduced by this vector.

### III. Introduction of a vector for introduction of recognition sequence (pTSspsRScodN) or a vector for introduction of recombinase gene (pTShygMRS35RRubipt) into Agrobacterium

*A. tumefaciens* 4404 strain was inoculated into 10 mL of a YEB liquid medium [5 g/L beef extract, 1 g/L yeast extract, 1 g/L peptone, 5 g/L sucrose, 2 mM MgSO₄, pH 7.2 at 22°C (hereinafter, pH at 22°C, unless otherwise specified)] and cultured at 28°C until OD630 reaches 0.4-0.6. After harvesting of this culture medium by centrifugation at 6,900 x g for 10 minutes at 4°C, the precipitated cells are suspended in 20 mL of 10 mM HEPES (pH 8.0) and the cells are collected again by centrifugation at 6,900 × g for 10 minutes at 4°C, and the obtained cells are suspended into 200 µL of a YEB liquid medium to be used as cell solution for plasmid introduction.

Subsequently, 50 µL, of this cell solution for plasmid introduction was mixed with 3 µL each of pTSspsRScodN and pTShygMRS35RRubipt which were prepared in I and II, respectively, in a 0.5 µL tube, and electroporation (Gene Pulser II System [BIORAD]) was conducted to introduce the above-described vector into A. *tumefaciens* 4404 strain. The cells after electroporation which 200 µL of the YEB liquid medium was supplemented, were cultured by shaking at 25°C for 1 hour, and were seeded into a 50 mg/L kanamycin-supplemented YEB agar medium (agar 1.5 w/v %, other compositions are the same as those described above) and cultured at 28°C for 2 days. As a result of this culture, cells which formed colonies were further introduced into the YEB liquid medium and cultured, and plasmids were extracted by the alkaline method, followed by digestion with restriction enzyme *Eco*RI or *Hin*dIII*,* and polyacrylamide gel electrophoresis was conducted to analyze the length of the restriction enzyme-digested products and demonstrated the introduction of pTSspsRScodN or pTShygMRS35RRubipt into *A. tumefaciens* 4404 strain. Cells into which the introduction of pTSspsRScodN was demonstrated were referred to as LBA4404 (pTSspsRScodN), and those into which the introduction of pTShygMRS35RRubipt was demonstrated as LBA4404 (pTShygMRS35RRubipt).

### IV. Introduction of a vector for introduction of recognition sequence (pTSspsRScodN) into Nicotiana tabacum

*Nicotiana tabacum* SR1 (hereinafter, the same below unless otherwise specified) grown in an incubator, whose medial veins were removed from its leaves, was cut into about 8 mm square to obtain 36 leaf discs, which were immersed into an LBA4404 (pTSspsRScodN) culture solution (OD630 = 0.25, after overnight culture in YEB liquid medium, culture concentration was adjusted by dilution with sterilized water) for about 1 minute for infection, and after removal of an excess culture solution on a sterile filter, were put on a 50 mg/L acetosyringone-supplemented MS agar medium without a plant hormone (hormone free) (T. Murashige and F. Skoog, Physiol. Plant., 15 : 473 (1962); however, agar was-supplemented at 0.8 w/v %) on upper sides of the leaves and cultured for 3 days at 25°C under full light (explant, plant tissue and plant individual were cultured under these conditions unless otherwise specified).

The above-described cultured leaf discs were introduced into MS agar medium supplemented with 6-benzylaminopurine (1 mg/L), naphthalene acetic acid (0.1 mg/L), carbenicillin (500 mg/L) and kanamycin (200 mg/L), and after the culture was continued, 24 adventitious buds were redifferentiated, and these adventitious buds separated were introduced to and further cultured on a hormone-free MS agar medium supplemented with carbenicillin (500 mg/L) and kanamycin (200 mg/L) for rooting, and 12 rooting individuals were obtained from 12 adventitious buds, and finally 12 lineages of kanamycin-resistant recombinants each derived from these 12 rooting individuals could be obtained.

Seeds were collected from acclimated rooting individuals for the obtained 12 lineages of kanamycin-resistant recombinants and seeded into a hormone free MS agar medium containing kanamycin (200 mg/L), and one kanamycin-resistant seedling strain, budded and grown, was each selected for one lineage.

### V. Analysis of Nicotiana tabacum into which a vector for introduction of recognition sequence (pTSspsRScodN) is introduced

Chromosomal DNA was extracted from the 12 strains of 12 kanamycin-resistant recombinant lineages, obtained in IV, by CTAB method, and was digested with restriction enzyme *Hind*III to be used for 0.8% agarose gel electrophoresis. The electrophoretic surface of the electrophoresed agarose gel after alkaline and acid treatment was transcribed to a nylon membrane and was hybridized with a probe with a sequence homologous to codA gene preliminarily labeled by DIG PCR labeling kit (purchased from Boehringer Mannheim). After hybridization, chemiluminescence detection was conducted with DIG Wash and Block Buffer (purchased from Boehringer Mannheim), and introduction of a copy of pTSspsRScodN was demonstrated in one lineage (Cod N-23) of the above-described 12 lineages.

### VI. Introduction of a vector for introduction of recombinase gene (pTShygMRS35RRubipt) into recognition sequence-introduced Nicotiana tabacum

In V, LBA4404 (pTShygMRS35RRubipt) was infected to recognition sequence-introduced *Nicotiana tabacum* Cod N-23, in which introduction of a copy of pTSspsRScodN was demonstrated, in the same manner as in IV. After infection, 64 leaf discs co-cultured with LBA4404 (pTShygMRS35RRubipt) for 3 days were introduced to an MS agar medium containing naphthalenacetic acid (1 mg/L), benzyladenine (0.1 mg/L), carbenicillin (500 mg/L) and hygromycin (20 mg/L) and the culture was continued to obtain 90 calluses, and these 90 calluses were introduced to mediums of the same compositions, followed by continuing the culture for 4 weeks, and 50 adventitious buds were obtained. When these adventitious buds were subcultured on a hormone-free MS agar medium, 24 rooting individuals could be obtained.

### VII. Analysis of Nicotiana tabacum into which a vector for introduction of recognition sequence (pTSspsRScodN) and a vector for introduction of recombinase gene (pTShygMRS35RRubipt) are introduced

### A. PCR analysis

Chromosomal DNA was extracted from 24 rooting individuals obtained in VI with Fast DNA Kit (BIO 101 Inc.), and PCR analysis was conducted using primer Km1 represented by SEQ ID NO:7 (5'-agaggctatt cggctatgca-3') and primer Km2 represented by SEQ ID NO:8 (5'-ccatgatatt cggcaagcag-3') which bind to the NPTII structural gene, primer RBS1 represented by SEQ ID NO:9 (5'-actgatagtt taaactgaag gcggg-3') which binds to the proximity of the right border of pTSspsRScodN and primer SPR 1a represented by SEQ ID NO: 10 (5'-atggcgttta tttattctgc-3') which binds to the sequence derived from spsU of pTSspsRScodN, primer SPL la represented by SEQ ID NO:11 (5'-acataagatg atacgcaagc-3') which binds to the sequence derived from spsL of pTSspsRScodN and primer pBSPh02 represented by SEQ ID NO:12 (5'-aagccggcga acgtggcgag aa-3') which binds to the proximity of the left border of pTSspsRScodN and primer Hm1 represented by SEQ ID NO:13 (5'-cgtctgtcga gaagtttctg-3') and primer Hm2 represented by SEQ ID NO: 14 (5'-ctatcggcga gtacttctac-3') which bind to the *Hpt* gene.

PCR reaction was conducted with 1 µg of DNA, dissolved in the mixture of 0.2 µM each of the primers, 10 mM Tris-HCl (pH 8.8 at 25°C), 50 mM KCI, 1.5 mM MgCl₂, 1 w/v% Triton X-100, 0.1 mM dNTPs, 1.25 units of Taq polymerase (purchased from CETUS) and 50 µL of the PCR buffer solution (after heating at 94°C for 1.5 minutes, 30 cycles of 94°C for 30 seconds, 60°C for 30 seconds and 72°C for 2 minutes). The PCR reaction solution was used for agarose gel electrophoresis to detect amplified DNA fragments.

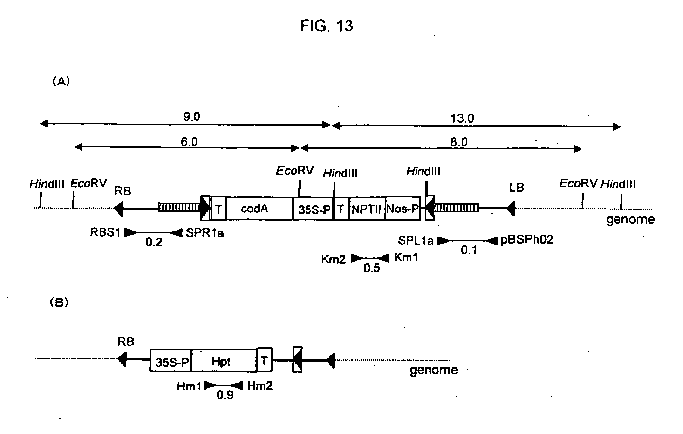

Here, when the *Nicotiana tabacum* used in the PCR analysis has chromosomes in which the structure derived from pTSspsRScodN is retained, DNA fragments of approximately 500 bp (Km1-Km2), approximately 200 bp (RBS1-SPR1a) and approximately 100 bp (SPL1a-pBSPh02) will be amplified (Fig. 13A). On the other hand, when the structure derived from pTSspsRScodN is disappeared from chromosomes by the function of the recombinase derived from pTShygMRS35RRubipt, none of the above-described DNA fragments will be amplified. And in the case where this chromosome disappearance has occurred when pTShygMRS35RRubipt was incorporated into chromosomes, a DNA fragment of approximately 900 bp (Hm1-Hm2) alone will be amplified (Figs. 12 and 13B).

As a result of the PCR analysis, amplified DNA fragments of approximately 900 bp were detected for all the 24 individuals used for electrophoresis. Amplified DNA fragments of approximately 500 bp, 200 bp and 100 bp were detected in 20 individuals, while none of these 3 DNA fragments was amplified in 4 individuals (Nos. 30, 34, 42 and 53). Thus, these 4 individuals were selected as those with predetermined chromosomes were disappeared (hereinafter, simply referred to as chromosome-disappeared individuals), and subsequently the following experiment was conducted.

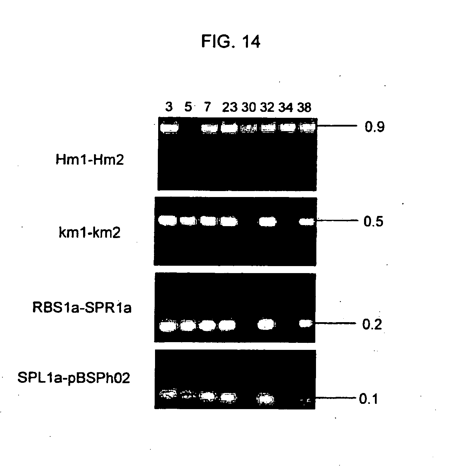

In Fig. 14, some results of agarose gel electrophoresis after the above-described PCR reaction are shown.

### B. Flow cytometry analysis on chromosome-disappeared individuals

The leaves of about 5 mm square were collected from the chromosome-disappeared individuals selected in A (Nos. 30, 34, 42 and 53) and immersed into solution A of the DNA reagent kit for plant analysis (Partec 06-5-4004), and after being immersed in this solution and shredded with a razor, 50 µL, of this was filtered through a CellTrics filter (Partec 06-4-2371), and the filtrate was collected. Using this filtrate to which 300 µL of solution B of the above-described reagent kit (Partec 06-5-4004) was added as a sample, flow cytometry analysis of chromosomal DNA amount was conducted using a ploidy Analyzer PA type (Partec). Here, the chromosomal DNA amount is detected as fluorescence intensity (FLI); the larger the chromosomal DNA amount is, the stronger the fluorescence intensity is indicated.

The results of the flow cytometry analysis are shown in Table 1.

**Table 1. Results of flow cytometry analysis on the Nicotiana tabacum SR1 chromosome-disappeared individuals, obtained in Example 1**

| Lineage | Amount of chromosomal DNA (fluorescence intensity) |
|---|---|
| 30 | 169.47 |
| 34 | 173.37 |
| 42 | 171.11 |
| 53 | 172.40 |
| Wild type | 179.98 |

As can be seen in Table 1, the DNA amounts of all the individuals, selected as chromosome-disappeared individuals in A (Nos. 30, 34, 42 and 53), are smaller than that of the wild type, suggesting that these are chromosome-disappeared individuals.

Specifically, in this example, chromosome-disappeared individuals can be easily produced with a probability of 4 individuals/64 leaf discs (= 6.3 × 10⁻²) from plant individuals to which a vector for introduction of a recombinase gene was introduced; in addition, the production was easy, which could be conducted without proceeding through germ cells and required only use of a vector with a very simple structure, very common gene transfer technique and plant tissue culturing technique.

### Example 2

### I. Construction of a vector for introduction of recombinase gene (pTL7MRS35RRubipt)

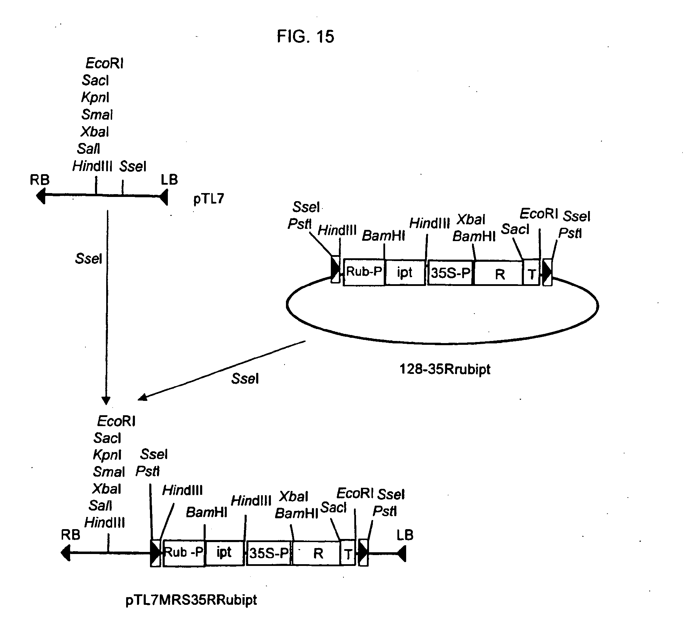

The RSs and the region therebetween are excised from the 128-35Rrubipt, constructed in Example 1-II, with restriction enzyme *Sse*I, was linked with the restriction enzyme *Sse*I site of the pTL7 (H. Ebinuma et al., Molecular Method of Plant Analysis, 22: 95 (2002)) to construct a vector plasmid, pTL7MRS35RRubipt, for the introduction of recombinase gene [Fig. 15: domestically deposited to the International Patent Organism Depositary, National Institute of Advanced Industrial Science and Technology (Address: Central 6, 1-1-1 Higashi, Tsukuba, Ibaraki 305-8566 Japan) on May 11, 2005 (Deposit No. FERM P-20533) or internationally deposited on December 8, 2005]. When this vector is introduced into a plant cell, the region between the RB and the LB derived from the T-DNA, indicated by the black triangles in the figure, is introduced into the plant cells in the same manner as in the vector for introduction of recognition sequence, pTSspsRScodN, and the vector for introduction of recombinase gene, pTShygMRS35RRubipt, constructed in Example 1.

When this vector for introduction of recombinase gene, pTL7MRS35RRubipt, is introduced into a plant cell to which the above-described vector for introduction of recognition sequence, pTSspsRScodN, was introduced, and the cells are cultured and grown, the two chromosomes produced by duplication, having two RSs derived from pTSspsRScodN, oriented in the opposite direction, cause DNA recombination in the same manner as in Example 1; in the case of asymmetric recombination, daughter cells are produced, in which the structures derived from the above-described pTSspsRScodN (i.e., two RSs oriented in the opposite direction and chromosomes having the codA gene and others which exist in the region between these two RSs) are lost, and in the case of symmetric recombination, a chromosome with these structures is maintained. Therefore, when pTL7MRS35RRubipt is introduced into a plant cell to which pTSspsRScodN has been introduced and the cells are cultured in the presence of 5-FC, cells in which symmetric recombination has been caused are killed by the function of the codA gene which remains in the chromosomes, and only cells in which chromosomes are disappeared by asymmetric recombination can be selected.

The R gene and *ipt* gene positioned in pTL7MRS35RRubipt for the introduction of the recombinase gene functions in the same manner as in Example 1. Specifically, when the R gene is incorporated into the chromosomes of plant cells, after a recombinase is produced to some extent, the R gene is disappeared and freed together with the *ipt* gene from the chromosomes to which they are introduced by the function of the recombinase produced by the gene itself, and then disappears. When the R gene is not disappeared from chromosomes through the above-described mechanism, the *ipt* gene functions as a negative selectable marker gene to eliminate relevant cells.

### II. Introduction of a vector for introduction of recombinase gene (pTL7MRS35RRubipt) into Agrobacterium

The pTL7MRS35RRubipt for the introduction of a recombinase gene, constructed in the above I, was introduced into *A. tumefaciens* 4404 strain in the same manner as in Example 1-III, and after DNA analysis to demonstrate this, cells were referred to as LBA4404 (pTL7MRS35RRubipt).

### III. Introduction of a vector for introduction of recombinase gene (pTL7MRS35RRubipt) into recognition sequence-introduced Nicotiana tabacum

The LBA4404 (pTL7MRS35RRubipt), obtained in the above-described II, was infected to the recognition sequence-introduced *Nicotiana tabacum* Cod N-23, in which introduction of a copy of pTSspsRScodN was demonstrated in Example 1-V, to introduce a vector for introduction of a recombinase gene (pTL7MRS35RRubipt) into recognition sequence-introduced *Nicotiana tabacum.* The infection was conducted in the same manner as in Example 1-IV.

After infection, 32 leaf discs co-cultured with LBA4404 (pTL7MRS35RRubipt) for 3 days were introduced to an MS agar medium containing 6-benzylaminopurine (1 mg/L), naphthalenacetic acid (0.1 mg/L) and carbenicillin (500 mg/L) and cultured for 1 week, and subsequently were introduced to the MS agar medium containing 6-benzylaminopurine (1 mg/L), naphthalenacetic acid (0.1 mg/L), carbenicillin (500 mg/L) and 5-FC (0.2 mg/L), and the culture was continued to obtain 24 calluses, and these 24 calluses were introduced to mediums of the same compositions, followed by continuing the culture, and 16 adventitious buds derived from 16 calluses were obtained. When these adventitious buds were subcultured on a hormone-free MS agar medium, 6 rooting individuals could be obtained.

### IV. Analysis of Nicotiana tabacum into which a vector for introduction of recognition sequence (pTSspsRScodN) and a vector for introduction of recombinase gene (pTL7MRS35RRubipt) are introduced

### A. PCR analysis

Chromosomal DNA was extracted from 6 rooting individuals obtained in III with Fast DNA Kit (BIO 101 Inc.), and PCR analysis was conducted using primers Km1 and Km2, primers RBS1 and SPR1a, primers SPL1a and pBSPh02, primer RBS2 represented by SEQ ID NO : 15 (5'-aaacgacaat ctgatcatga gcgga-3') which binds to the T-DNA of the right border of pTL7MRS35RRubipt and primer Lza9 represented by SEQ ID NO:16 (5'-ggctcgtatg ttgtgtggaa ttgt-3'), primer Ra represented by SEQ ID NO:17 (5'-ccaaggatac tgaaatttca acaat-3') which binds to the R gene of pTL7MRS35RRubipt and primer Rc represented by SEQ ID NO:18 (5'-ttatttgaaa gatatgaagc tgtca-3'), and primer OMT represented by SEQ ID NO:19 (5'-taagacctat atccacttca aaaca-3') and primer OMT3 represented by SEQ ID NO:20 (5'-agctagccct gtggcgtgcc ttcca-3') which bind to the O-methyltransferase (OMT) gene of the genome of *Nicotiana tabacum,* and PCR analysis was conducted under the same conditions as those in Example 1.

Here, when the *Nicotiana tabacum* used in the PCR analysis has chromosomes in which the structure derived from pTSspsRScodN is retained, DNA fragments of approximately 500 bp (Km1-Km2), approximately 200 bp (RBS1-SPR1a) and approximately 100 bp (SPL1a-pBSPh02) will be amplified (Fig. 13A). On the other hand, when the structure derived from pTSspsRScodN is disappeared from chromosomes by the function of the recombinase derived from pTL7MRS35RRubipt, none of the above-described DNA fragments will be amplified.

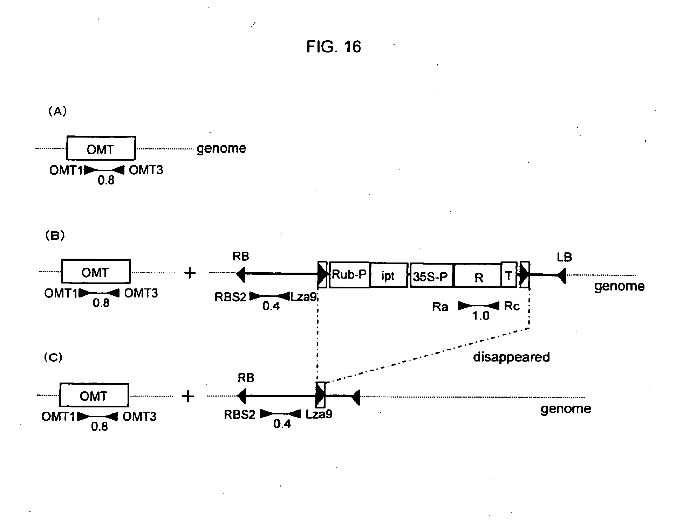

When this chromosome disappearance occurs without incorporation of pTL7MRS35RRubipt into chromosomes, a DNA fragment of approximately 800 bp (OMT1-OMT3) alone will be amplified (Fig. 16A). In the case where this chromosome disappearance has occurred when pTL7MRS35RRubipt was incorporated into chromosomes, DNA fragments of approximately 800 bp (OMT1-OMT3) and approximately 400 bp (RBS2-Lza9), or approximately 800 bp (OMT1-OMT3), approximately 400 bp (RBS2-Lza9) and approximately 1.0 kb (Ra-Rc) will be amplified. Specifically, amplification of DNA fragments of approximately 800 bp (OMT1-OMT3), and approximately 400 bp (RBS2-Lza9) indicate that the structure derived from pTL7MRS35RRubipt, once incorporated into chromosomes, was subsequently disappeared by the function of a recombinase (Fig. 16C), while amplification of DNA fragments of approximately 800 bp (OMT1-OMT3), approximately 400 bp (RBS2-Lza9) and approximately 1.0 kb indicate that the structure derived from pTL7MRS35RRubipt, incorporated into chromosomes, was not disappeared subsequently but maintained in chromosomes (Fig. 16B).

As a result of the PCR analysis, an amplified DNA fragment of approximately 800 bp was detected for all 6 individuals used for electrophoresis, while none of the amplified DNA fragments of approximately 500 bp, 200 bp, 100 bp and 1.0 kbp were detected. The amplified DNA fragment of approximately 400 bp was detected in 4 of the 6 individuals (Nos. 1, 2, 4 and 7), but was not detected for the other 2 individuals (Nos. 8 and 12).

The above results suggest that all these 6 individuals are chromosome-disappeared individuals, of which two individuals (Nos. 8 and 12) are individuals in which chromosome disappearance occurred without pTL7MRS35RRubipt being incorporated, i.e., individuals derived from cells in which, when pTL7MRS35RRubipt was introduced in the above-described III, the R gene positioned in it was not incorporated into chromosomes but remained in a free state to be expressed, and as a result, chromosome disappearance occurred; other four individuals (Nos. 1, 2, 4 and 7) are individuals in which chromosome disappearance occurred without pTL7MRS35RRubipt being incorporated, i.e., individuals derived from cells in which, when pTL7MRS35RRubipt was introduced in the above-described III, the R gene positioned in it was also incorporated into chromosomes, and as a result, chromosome disappearance occurred. In all four individuals in which incorporation of pTL7MRS35RRubipt into chromosomes caused chromosome disappearance, after incorporation of this pTL7MRS35RRubipt, the structure derived from pTL7MRS35RRubipt, such as the R gene, was considered to be disappeared by the function of the recombinase produced by R gene expression.

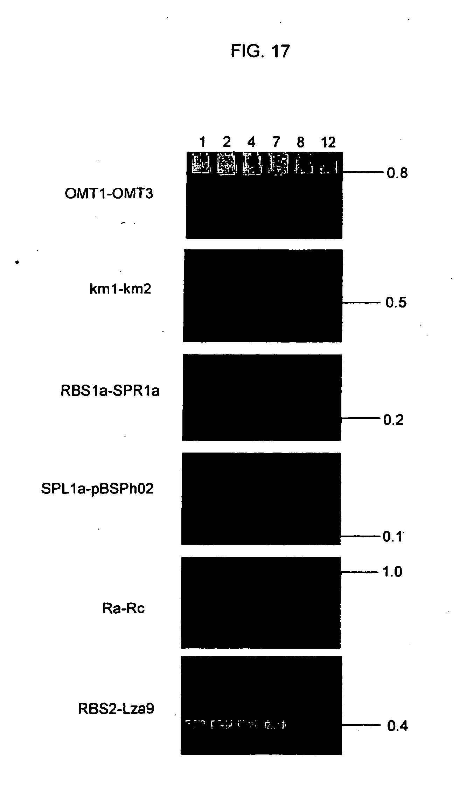

In Fig. 17, some results of agarose gel electrophoresis after the above-described PCR reaction are shown.

### B. Flow cytometry analysis on chromosome-disappeared individuals

Flow cytometry analysis was conducted on the above-described 6 individuals (Nos. 1, 2, 4, 7, 8 and 12), determined as chromosome-disappeared individuals in A, in the same manner as in VI-B of Example 1.

The results of the flow cytometry analysis are shown in Table 2.

**Table 2. Results of flow cytometry analysis on the Nicotiana tabacum SR1 chromosome-disappeared individuals, obtained in Example 2**

| Lineage | Amount of chromosomal DNA (fluorescence intensity) |
|---|---|
| 1 | 174.75 |
| 2 | 171.56 |
| 4 | 169.12 |
| 7 | 172.58 |
| 8 | 174.19 |
| 12 | 171.76 |
| Wild type | 181.45 |

As can be seen in Table 2, the DNA amounts of all the six individuals, demonstrated as chromosome-disappeared individuals in A (Nos. 1, 2, 4, 7, 8 and 12), are smaller than that of the wild type, suggesting that these are chromosome-disappeared individuals.

Specifically, in this example, chromosome-disappeared individuals could be produced with a probability of 6 individuals/32 leaf discs (= 1.9 × 10⁻¹), from plant individuals to which a vector for introduction of a recombinase gene was introduced; furthermore, in the introduction of a vector for introduction of a recombinase, only chromosome-disappeared individuals could be obtained as rooting individuals by culturing tissues after the introduction in the presence of 5-FC. Two of the six (33%) produced chromosome-disappeared individuals, are individuals produced without a recombinase gene being incorporated, and had no trace of the vector for introduction of a recombinase gene, as well as no trace of the vector for introduction of a recognition sequence, left in the cells (i.e., individuals without trace of genetic manipulation being left).

The production was easy, which could be conducted without proceeding through germ cells and required only use of a vector with very simple structure, very common gene transfer technique and plant tissue culturing technique.

### Example 3

### 1. Construction of a vector for introduction of recombinase gene (pTL735R)

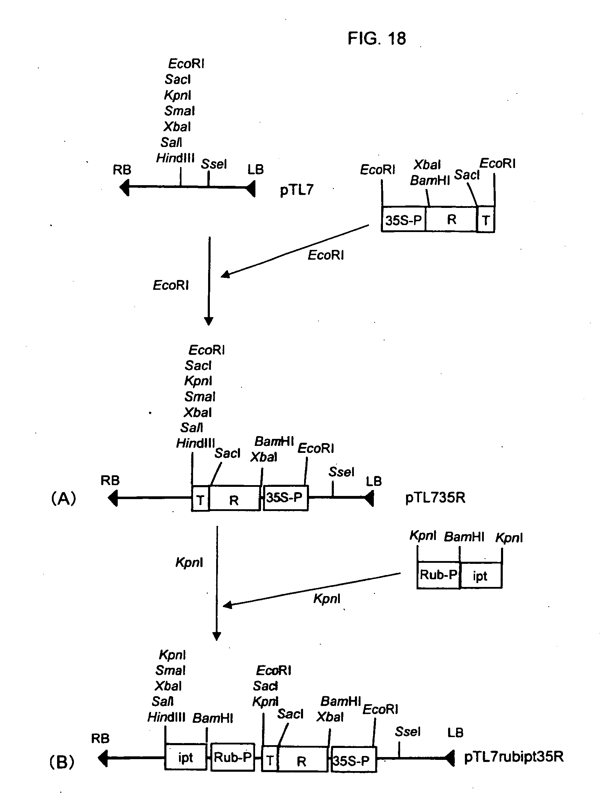

A plasmid vector for introduction of recombinase gene, pTL735R, was constructed by linking the R structural gene linked with 35S-P and the polyadenylation signal to the EcoRI site of pTL7 (Fig. 18A), and was internationally deposited to the International Patent Organism Depositary, National Institute of Advanced Industrial Science and Technology (Address: Central 6, 1-1-1 Higashi, Tsukuba, Ibaraki 305-8566 Japan) on December 8, 2005 (Deposit No. FERM ABP-10472). When this vector is introduced into a plant cell, the region between the RB and the LB derived from the T-DNA, indicated by the black triangles in the figure, is introduced into chromosomes in the same manner as for pTSspsRScodN for the introduction of a recognition sequence and pTShygMRS35RRubipt for a recombinase gene, constructed in Example 1, and pTL7MRS35RRubipt for the introduction of a recombinase gene, constructed in Example 2.

When the vector for introduction of a recombinase gene, pTL735R, is introduced into a plant cell to which the above-described vector for introduction of a recognition sequence, pTSspsRScodN, was introduced, and the cells are cultured and grown, DNA recombination occurs between the two chromosomes, having two RSs derived from pTSspsRScodN, oriented in the opposite direction; in the case of asymmetric recombination, daughter cells are produced, in which the structures derived from this pTSspsRScodN (i.e., two RSs oriented in the opposite direction and chromosomes having the codA gene and others which exist in the region between these two RSs) are lost, and in the case of symmetric recombination, a chromosome with these structures is maintained. Therefore, when pTL735R is introduced into a plant cell to which pTSspsRScodN has been introduced and the cells are cultured in the presence of 5-FC, cells in which symmetric recombination has been caused are killed by the function of the codA gene which remains in the chromosomes, and only cells in which chromosomes are disappeared by asymmetric recombination can be selected.

The R gene positioned in pTL735R for the introduction of a recombinase gene, introduced into a plant cell, is expressed regardless of whether being incorporated into the chromosomes of the cells; however, when the R gene is expressed without being incorporated into chromosomes, the recombinase functions transiently, and the chromosomes are disappeared by the above-described mechanism, furthermore, cells with no trace of the vector for introduction of recombinase gene, pTL735R, as well as no trace of the vector for introduction of recombinase, pTSspsRScodN, left in the cells, can be obtained; thus, in this example, the function of the free R gene is utilized.

### II Introduction of a vector for introduction of recombinase gene (pTL735R) into Agrobacterium

The vector for introduction of recombinase gene, pTL735R, constructed in the above I, was introduced into *A. tumefaciens* 4404 strain in the same manner as in Example 1-III, and after DNA analysis to demonstrate this, these cells were referred to as LBA4404 (pTL735R).

### III. Introduction of a vector for introduction of recombinase gene (pTL735R) into recognition sequence-introduced Nicotiana tabacum

The LBA4404 (pTL735R), obtained in the above-described II, was infected to the recognition sequence-introduced *Nicotiana tabacum* Cod N-23, in which introduction of a copy of pTSspsRScodN was demonstrated, to introduce a vector for introduction of a recombinase gene (pTL735R) into the recognition sequence-introduced *Nicotiana tabacum.* The infection was conducted in the same manner as in Example 1-IV.

After infection, 32 leaf discs co-cultured with LBA4404 (pTL735R) for 3 days were introduced to an MS agar medium containing 6-benzylaminopurine (1 mg/L), naphthalenacetic acid (0.1 mg/L) and carbenicillin (500 mg/L) and cultured for 1 week, and subsequently were introduced to an MS agar medium containing 6-benzylaminopurine (1 mg/L), naphthalenacetic acid (0.1 mg/L), carbenicillin (500 mg/L) and 5-FC (0.2 mg/L), and culture was continued to obtain 19 calluses, and these 19 calluses were introduced to mediums of the same compositions, followed by continuing the culture, and 14 adventitious buds derived from 14 calluses were obtained. When these adventitious buds were subcultured on a hormone-free MS agar medium, 14 rooting individuals could be obtained.

### IV. Analysis of Nicotiana tabacum into which a vector for introduction of recognition sequence (pTSspsRScodN) and a vector for introduction of recombinase gene (pTL735R) are introduced

### A. PCR analysis

Chromosomal DNA was extracted from the 14 rooting individuals, obtained in III, with a Fast DNA Kit (BIO 101 Inc.), and PCR analysis was conducted using primers Km1 and Km2, primers RBS1 and SPR1a, primers SPL1a and pBSPh02, used in Examples 1 and 2, and primers Ra and Rc and primers OMT1 and OMT3, used in Example 2, and PCR analysis was conducted under the same conditions as those in Example 1.

Here, when the *Nicotiana tabacum* used in the PCR analysis has chromosomes in which the structure derived from pTSspsRScodN is retained, DNA fragments of approximately 500 bp (Km1-Km2), approximately 200 bp (RBS1-SPR1a) and approximately 100 bp (SPL1a-pBSPh02) will be amplified (Fig. 13A). On the other hand, when the structure derived from pTSspsRScodN is disappeared from chromosomes by the function of the recombinase derived from pTL736R, none of the DNA fragments will be amplified.

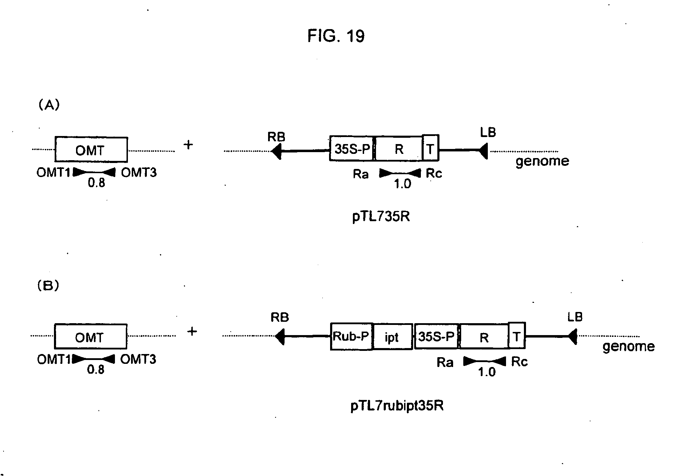

When this chromosome disappearance occurs without incorporation of pTL736R into chromosomes, a DNA fragment of approximately 800 bp (OMT1-OMT3) alone will be amplified (Fig. 16A). When this chromosome disappearance has occurred with incorporation of pTL735R into chromosomes, DNA fragments of approximately 800 bp (OMT1-OMT3) and 1.0 kb (Ra-Rc) will be amplified (Fig. 19A).

As a result of the PCR analysis, an amplified DNA fragment of approximately 800 bp was detected for all 14 individuals (R-1, 2, 3, 4, 5, 6, 7, 8, 9, 10, 11, 12, 13 and 15) used for electrophoresis. In 11 individuals (R-1, 2, 3, 4, 5, 6, 7, 9, 10, 11 and 15) of these, none of the amplified DNA fragments of approximately 500 bp, 200 bp and 100 bp were detected; in addition, the amplified DNA fragment of approximately 1.0 kb was detected in 6 individuals (R-3, 5, 7, 9, 11 and 15), but was not detected in 5 individuals (R-1, 2, 4, 6 and 10).

Thus, in this example, 11 chromosome-disappeared individuals were obtained, of which 5 individuals (R-1, 2, 4, 6 and 10) are considered to be individuals in which chromosome disappearance occurred without pTL735R being incorporated, i.e., individuals derived from cells in which, when pTL735R was introduced in the above-described III, the R gene positioned in it was not incorporated into chromosomes but remained in a free state to be expressed, and as a result, chromosome disappearance occurred.

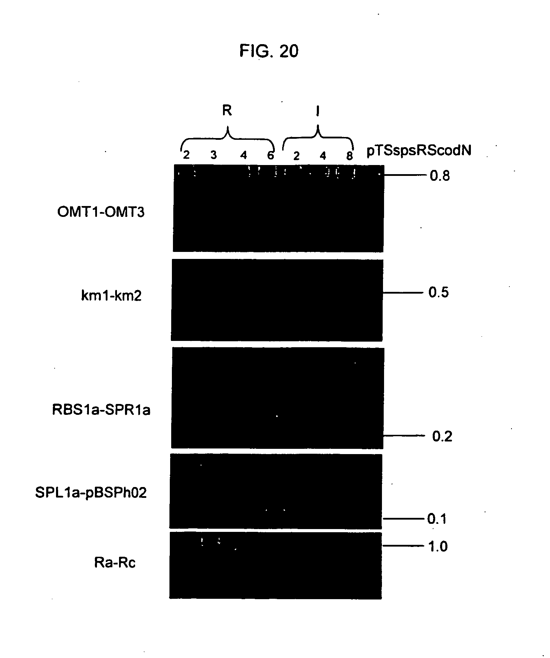

In Fig. 20, some results of agarose gel electrophoresis after the above-described PCR reaction are shown.

### B. Flow cytometry analysis on chromosome-disappeared individuals

Flow cytometry analysis was conducted on the above-described 5 individuals (R-1, 2, 4, 6 and 10) of the 11 individuals, determined as chromosome-disappeared individuals in A, in the same manner as in VI-B of Example 1.

The results are shown in Table 3.

**Table 3. Results of flow cytometry analysis on the Nicotiana tabacum SR1 chromosome-disappeared individuals, obtained in Examples 3 and 4**

| Lineage | Amount of chromosomal DNA (fluorescence intensity) |
|---|---|
| R-1 | 173.75 |
| R-2 | 168.98 |
| R-4 | 172.77 |
| R-6 | 174.24 |
| R-10 | 173.21 |
| I-2 | 171.96 |
| I-4 | 170.59 |
| I-8 | 169.28 |
| I-10 | 173.19 |
| I-11 | 170.79 |
| Wild type | 181.45 |

As can be seen in Table 3, the DNA amounts of all five individuals (R-1, 2, 4, 6 and 10), are smaller than that of the wild type, suggesting these are chromosome-disappeared individuals.

Specifically, in this example, chromosome-disappeared individuals could be produced without incorporation of a recombinase gene into the chromosomes with a probability of 5 individuals/32 leaf discs (= 1.6 x 10⁻¹), from plant individuals to which a vector for introduction of a recombinase gene was introduced; chromosome-disappeared individuals could be obtained with no trace of the vector for introduction of a recombinase gene, as well as no trace of the vector for introduction of a recognition sequence, left in the cells (i.e., individuals without trace of genetic manipulation being left).

The production was easy, which could be conducted without proceeding through germ cells and required only use of a vector with a very simple structure, very common gene transfer technique and plant tissue culturing technique.

### Example 4

### 1. Construction of a vector for introduction of recombinase gene (pTL7rubipt35R)

A plasmid vector for introduction of recombinase gene, pTL7rubipt35R, was constructed by linking the *ipt* gene linked with Rub-P to the KpnI site of pTL735R (Fig. 18B), and was internationally deposited to the International Patent Organism Depositary, National Institute of Advanced Industrial Science and Technology (Address: Central 6, 1-1-1 Higashi, Tsukuba, Ibaraki 305-8566 Japan) on December 8, 2005 (Deposit No. FERM ABP-10473). When this vector is introduced into a plant cell, the region between the RB and the LB derived from the T-DNA, indicated by the black triangles in the figure, is introduced into chromosomes in the same manner as for pTSspsRScodN for the introduction of a recognition sequence and pTShygMRS35RRubipt for a recombinase gene, constructed in Example 1, pTL7MRS35RRubipt for the introduction of a recombinase gene, constructed in Example 2, and pTL735R for the introduction of a recombinase gene, constructed in Example 3.

When this vector for introduction of recombinase gene, pTL7rubipt35R, is introduced into a plant cell to which the above-described vector for introduction of recognition sequence, pTSspsRScodN, was introduced, and the cells are cultured and grown, DNA recombination occurs between the two chromosomes, produced by duplication, having two RSs derived from pTSspsRScodN, oriented in the opposite direction, in the same manner as in Examples 1, 2 and 3; in the case of asymmetric recombination, daughter cells are produced, in which the structures derived from this pTSspsRScodN (i.e., chromosome having two RSs oriented in the opposite direction and the codA gene and others which exist in the region between these two RSs) are lost, and in the case of symmetric recombination, a chromosome with these structures is maintained. Therefore, when pTL7rubipt35R is introduced into a plant cell to which pTSspsRScodN has been introduced and the cells are cultured in the presence of 5-FC, cells in which symmetric recombination has been caused are killed by the function of the codA gene which remains in the chromosomes, and only cells in which chromosomes are disappeared by asymmetric recombination can be selected.

The R gene positioned in the vector for introduction of recombinase gene, pTL7rubipt35R introduced into a plant cell, is expressed regardless of whether being incorporated into the chromosomes of the cells, in the same manner as for the R gene positioned in the vector for introduction of recombinase gene, pTL735R constructed in Example 3; when the R gene is expressed without being incorporated into chromosomes, the recombinase functions transiently, and the chromosomes is disappeared by the above-described mechanism, furthermore, cells with no trace of the vector for introduction of the recombinase gene (pTL7rubipt35R), as well as no trace of the vector for introduction of the recognition sequence (pTSspsRScodN), left in the cells, can be obtained.

However, the *ipt* gene is also positioned in the vector for introduction of the recombinase gene (pTL7rubipt35R), constructed in this example, as a negative selectable marker gene together with the R gene and thus introduction of pTL7rubipt35R into a plant cell inhibits normal growth and differentiation of cells having this chromosome since the *ipt* gene is also incorporated into this chromosome and expressed even if the R gene positioned in this vector is incorporated into the chromosomes of the cells. The cells are thus disappeared, and cells in which the recombinase has been allowed to act transiently can be reliably obtained.

### II. Introduction of a vector for introduction of recombinase gene (pTL7rubipt35R) into Agrobacterium

The pTL7rubipt35R for the introduction of the recombinase gene, constructed in the above I, was introduced into *A. tumefaciens* 4404 strain in the same manner as in Example 1-III, and after DNA analysis to demonstrate this, the strain was referred to as LBA4404 (pTL7rubipt35R).

### III. Introduction of a vector for introduction of recombinase gene (pTL7rubipt35R) into recognition sequence-introduced Nicotiana tabacum

The LBA4404 (pTL7rubipt35R), obtained in the above-described II, was infected to the recognition sequence-introduced *Nicotiana tabacum* Cod N-23, in which introduction of a copy of pTSspsRScodN was demonstrated in Example 1-V, to introduce a vector for introduction of the recombinase gene (pTL7rubipt35R) into the recognition sequence-introduced *Nicotiana tabacum.* The infection was conducted in the same manner as in Example 1-IV.

After infection, 32 leaf discs co-cultured with LBA4404 (pTL7rubipt35R) for 3 days were introduced to an MS agar medium containing 6-benzylaminopurine (1 mg/L), naphthalenacetic acid (0.1 mg/L) and carbenicillin (500 mg/L) and cultured for 1 week, and subsequently were introduced to an MS agar medium containing 6-benzylaminopurine (1 mg/L), naphthalenacetic acid (0.1 mg/L), carbenicillin (500 mg/L) and 5-FC (0.2 mg/L), and the culture was continued to obtain 53 calluses, and these 53 calluses were introduced to mediums of the same compositions, followed by continuing the culture, and 5 adventitious buds derived from 5 calluses were obtained. When these adventitious buds were subcultured on a hormone-free MS agar medium, 5 rooting individuals could be obtained.

### IV. Analysis of Nicotiana tabacum into which a vector for introduction of recognition sequence (pTSspsRScodN) and a vector for introduction of recombinase gene (pTL7rubipt35R) are introduced

### A. PCR analysis

Chromosomal DNA was extracted from the 5 rooting individuals, obtained in III, in the same manner as Example 3, with a Fast DNA Kit (BIO 101 Inc.), and PCR analysis was conducted using primers Km1 and Km2, primers RBS1 and SPR1a, and primers SPL1a and pBSPh02, used in Examples 1 and 2, and primers Ra and Rc and primers OMT1 and OMT3, used in Example 2, and PCR analysis was conducted under the same conditions as those in Example 1.

Therefore, when the *Nicotiana tabacum* used in the PCR analysis has chromosomes in which the structure derived from pTSspsRScodN is retained, DNA fragments of approximately 500 bp (Km1-Km2), approximately 200 bp (RBS1-SPR1a) and approximately 100 bp (SPL1a-pBSPh02) will be amplified (Fig. 13A). On the other hand, when the chromosome to which the structure derived from the pTSspsRScodN is introduced is disappeared by the function of the recombinase derived from pTL7rubipt35R, none of the above-described DNA fragments will be amplified.

When this chromosome disappearance occurs without incorporation of pTL7rubipt35R into chromosomes, a DNA fragment of approximately 800 bp (OMT1-OMT3) alone will be amplified (Fig. 16A). When this chromosome disappearance has occurred with incorporation of pTL7rubipt35R into chromosomes, DNA fragments of approximately 800 bp (OMT1-OMT3) and 1.0 kb (Ra-Rc) will be amplified (Fig. 19B).

As a result of the PCR analysis, an amplified DNA fragment of approximately 800 bp was detected for all 5 individuals (I-2, 4, 8, 10 and 11) used for electrophoresis, while amplified DNA fragments of approximately 500 bp, 200 bp, 100 bp and 1.0 kb were not detected.

Thus, in this example, 5 chromosome-disappeared individuals were obtained (I-2, 4, 8, 10 and 11), all of which are considered to be individuals in which chromosome disappearance occurred without pTL7rubipt35R being incorporated, i.e., individuals derived from cells in which, when pTL7rubipt35R was introduced in the above-described III, the R gene positioned in it was not incorporated into chromosomes but remained in a free state to be expressed, and as a result, chromosome disappearance occurred.

In Fig. 20, some results of agarose gel electrophoresis after the above-described PCR reaction are shown.

### B. Flow cytometry analysis on chromosome-disappeared individuals

Flow cytometry analysis was conducted on the above-described 5 individuals (I-2, 4, 8, 10 and 11), determined as chromosome-disappeared individuals in A, in the same manner as in VI-B of Example 1.

The results are shown in Table 3.

As can be seen in Table 3, the DNA amounts of all the above-described 5 individuals (I-2, 4, 8, 10 and 11) are smaller than that of the wild type, suggesting these are chromosome-disappeared individuals.

Specifically, in this example, chromosome-disappeared individuals could be obtained with a probability of 5 individuals/32 leaf discs (= 1.6 × 10⁻¹), from plant individuals to which a vector for introduction of a recombinase gene was introduced. Furthermore, in this example, a negative selectable marker gene (*ipt* gene) positioned in the vector together with a recombinase gene inhibited the redifferentiation of cells, with a recombinase gene incorporated into the chromosomes, into rooting individuals and thus, in all the obtained chromosome-disappeared individuals, the chromosomes were disappeared without the recombinase gene being incorporated into the chromosomes, i.e., individuals with no trace of the vector for introduction of the recombinase gene, as well as no trace of the vector for introduction of the recognition sequence, left in the cells (i.e., individuals without trace of genetic manipulation being left).

The production was easy, which could be conducted without proceeding through germ cells and required only use of a vector with a very simple structure, very common gene transfer technique and plant tissue culturing technique.

### INDUSTRIAL APPLICABILITY

The present invention provides a simple aneuploid production process, applicable to all species, without producing unexpected damage to chromosomes other than a chromosome which is to be disappeared.

### Free text of sequence listings

SEQ ID NO:1 - Description of artificial sequence: Synthetic DNA
SEQ ID NO:2 - Description of artificial sequence: Synthetic DNA
SEQ ID NO:3 - Description of artificial sequence: Synthetic DNA
SEQ ID NO:4 - Description of artificial sequence: Synthetic DNA
SEQ ID NO:5 - Description of artificial sequence: Synthetic DNA
SEQ ID NO:6 - Description of artificial sequence: Synthetic DNA
SEQ ID NO:7 - Description of artificial sequence: Synthetic DNA
SEQ ID NO:8 - Description of artificial sequence: Synthetic DNA
SEQ ID NO:9 - Description of artificial sequence: Synthetic DNA
SEQ ID NO:10 - Description of artificial sequence: Synthetic DNA
SEQ ID NO:11 - Description of artificial sequence: Synthetic DNA
SEQ ID NO:12 - Description of artificial sequence: Synthetic DNA
SEQ ID NO:13 - Description of artificial sequence: Synthetic DNA
SEQ ID NO:14 - Description of artificial sequence: Synthetic DNA
SEQ ID NO:15 - Description of artificial sequence: Synthetic DNA
SEQ ID NO:16 - Description of artificial sequence: Synthetic DNA
SEQ ID NO:17 - Description of artificial sequence: Synthetic DNA
SEQ ID NO:18 - Description of artificial sequence: Synthetic DNA
SEQ ID NO:19 - Description of artificial sequence: Synthetic DNA
SEQ ID NO:20 - Description of artificial sequence: Synthetic DNA

## Claims

1. A process for producing a plant cell in which one or more chromosomes are disappeared, said process comprises the following steps (A)-(C):
(A) introducing a vector comprising:
two site-specific recombinase recognition sequences oriented in the opposite direction,
a site-specific recombinase gene which is positioned inside or outside of the region between the two site-specific recombinase recognition sequences and which encodes a site-specific recombinase which recognizes the two site-specific recombinase recognition sequences, and
a negative selectable marker gene positioned inside or outside of the region between the two site-specific recombinase recognition sequences oriented in the opposite direction,
into a plant cell;
(B) culturing and growing the vector-introduced plant cell (A); and
(C) selecting a cell in which a chromosome is disappeared, from the cell grown in (B),
wherein the plant cell is cultured under conditions where the negative selectable marker gene functions in the step (B).

2. A process for producing a plant cell in which one or more chromosomes are disappeared, said process comprising the following steps (A)-(C):
(A) introducing a vector comprising:
one site-specific recombinase recognition sequence comprising a point-symmetric nucleotide sequence,
a site-specific recombinase gene encoding a site-specific recombinase which recognizes the site-specific recombinase recognition sequence, and
a negative selectable marker gene,
into a plant cell;
(B) culturing and growing the vector-introduced plant cell in (A); and
(C) selecting a cell in which a chromosome is disappeared, from the cell grown in (B),
wherein the plant cell is cultured under conditions where the negative selectable marker gene functions in the step (B).

3. A process for producing a plant cell in which one or more chromosomes are disappeared, said process comprises the following steps (A)-(D):
(A) introducing
a vector comprising two site-specific recombinase recognition sequences oriented in the opposite direction, and a negative selectable marker gene positioned inside or outside of the region between the two site-specific recombinase recognition sequences, or
a vector comprising one site-specific recombinase recognition sequence comprising a point-symmetric nucleotide sequence, and a negative selectable marker gene,
into a plant cell;
(B) culturing the vector-introduced plant cell in (A) and allowing the site-specific recombinase which recognizes the two site-specific recombinase recognition sequences to act transiently in the cell during at least growth of the cell;
(C) culturing and growing a cell in which the site-specific recombinase has been allowed to act in (B); and
(D) selecting a cell in which a chromosome is disappeared, from the cell grown in (C)
wherein the plant cell is cultured under conditions where the negative selectable marker gene functions in the step (C).

4. The process for producing a plant cell in which one or more chromosomes are disappeared according to claim 3, wherein the site-specific recombinase is allowed to act transiently by introducing the site-specific recombinase into the cultured plant cell in the step (B).

5. The process for producing a plant cell in which one or more chromosomes are disappeared according to claim 3, wherein the site-specific recombinase is allowed to act transiently by introducing a site-specific recombinase gene encoding the site-specific recombinase into the cultured plant cell in the step (B).

6. The process for producing a plant cell in which one or more chromosomes are disappeared according to claim 5, wherein introduction of the site-specific recombinase gene is carried out by introducing a vector comprising a site-specific recombinase gene and a negative selectable marker gene, into a plant cell.

7. The process for producing a plant cell in which one or more chromosomes are disappeared according to claim 5,
wherein the site-specific recombinase gene is positioned between the two site-specific recombinase recognition sequences, oriented in the same direction, which are recognized by the site-specific recombinase encoded by the site-specific recombinase gene, in a vector, and
the vector is introduced into a plant cell.

8. The process for producing a plant cell in which one or more chromosomes are disappeared according to claim 7, wherein a negative selectable marker gene, together with a site-specific recombinase gene, is positioned between the two site-specific recombinase recognition sequences, oriented in the same direction, in the vector.

9. The process for producing a plant cell in which one or more chromosomes are disappeared according to any one of claims 1 to 3, 6 and 8, wherein a lethal induction gene or a morphological abnormality induction gene is used as the negative selectable marker gene.

## Patentansprüche

1. Verfahren zum Herstellen einer Pflanzenzelle, in welcher ein oder mehrere Chromosomen verschwunden sind, wobei das Verfahren umfasst die folgenden Schritte (A)-(C):
(A) Einführen eines Vektors, umfassend:
zwei ortsspezifische Rekombinase-Erkennungssequenzen, orientiert in der entgegengesetzten Richtung,
ein ortsspezifisches Rekombinase-Gen, welches innerhalb oder außerhalb der Region zwischen den zwei ortsspezifischen Rekombinase-Erkennungssequenzen positioniert ist und welches eine ortsspezifische Rekombinase codiert, welche die zwei ortsspezifischen Rekombinase-Erkennungssequenzen erkennt, und
ein negativ selektierbares Marker-Gen, positioniert innerhalb oder außerhalb der Region zwischen den zwei ortsspezifischen Rekombinase-Erkennungssequenzen, orientiert in der entgegengesetzten Richtung,
in eine Pflanzenzelle;
(B) Kultivieren und Züchten der Pflanzenzelle mit dem in (A) eingeführten Vektor; und
(C) Selektieren einer Zelle, in welcher ein Chromosom verschwunden ist, aus der Zelle, gezüchtet in (B),
wobei die Pflanzenzelle unter Bedingungen kultiviert wird, wo das negativ selektierbare Marker-Gen im Schritt (B) funktioniert.

2. Verfahren zum Herstellen einer Pflanzenzelle, in welcher ein oder mehrere Chromosomen verschwunden sind, wobei das Verfahren die folgenden Schritte (A)-(C) umfasst:
(A) Einführen eines Vektors, umfassend:
eine ortsspezifische Rekombinase-Erkennungssequenz, umfassend eine Punkt-symmetrische Nucleotidsequenz,
ein ortsspezifisches Rekombinase-Gen, codierend eine ortsspezifische Rekombinase, welche die ortsspezifische Rekombinase-Erkennungssequenz erkennt, und
ein negativ selektierbares Marker-Gen,
in eine Pflanzenzelle;
(B) Kultivieren und Züchten der Pflanzenzelle mit dem in (A) eingeführten Vektor; und
(C) Selektieren einer Zelle, in welcher ein Chromosom verschwunden ist, aus der Zelle, gezüchtet in (B),
wobei die Pflanzenzelle unter Bedingungen kultiviert wird, wo das negativ selektierbare Marker-Gen im Schritt (B) funktioniert.

3. Verfahren zum Herstellen einer Pflanzenzelle, in welcher ein oder mehrere Chromosomen verschwunden sind, das Verfahren umfasst die folgenden Schritte (A)-(D):
(A) Einführen eines Vektors, umfassend zwei ortsspezifische Rekombinase-Erkennungssequenzen, orientiert in der entgegengesetzten Richtung, und ein negativ selektierbares Marker-Gen, positioniert innerhalb oder außerhalb der Region zwischen den zwei ortsspezifischen Rekombinase-Erkennungssequenzen, oder
eines Vektors, umfassend eine ortsspezifische Rekombinase-Erkennungssequenz, umfassend eine Punkt-symmetrische Nucleotidsequenz, und ein negativ selektierbares Marker-Gen,
in eine Pflanzenzelle;
(B) Kultivieren der Pflanzenzelle mit dem in (A) eingeführten Vektor und Erlauben, dass die ortsspezifische Rekombinase, welche die zwei ortsspezifischen Rekombinase-Erkennungssequenzen erkennt, zumindest während des Wachstums der Zelle vorübergehend in der Zelle wirkt;
(C) Kultivieren und Züchten einer Zelle, in welcher der ortsspezifischen Rekombinase erlaubt worden ist, in (B) zu wirken; und
(D) Selektieren einer Zelle, in welcher ein Chromosom verschwunden ist, aus der Zelle, gezüchtet in (C),
wobei die Pflanzenzelle unter Bedingungen kultiviert wird, wo das negativ selektierbare Marker-Gen im Schritt (C) funktioniert.

4. Verfahren zum Herstellen einer Pflanzenzelle, in welcher ein oder mehrere Chromosomen verschwunden sind, gemäß Anspruch 3, wobei der ortsspezifischen Rekombinase erlaubt wird, vorübergehend zu wirken, indem die ortsspezifische Rekombinase im Schritt (B) in die kultivierte Pflanzenzelle eingeführt wird.

5. Verfahren zum Herstellen einer Pflanzenzelle, in welcher ein oder mehrere Chromosomen verschwunden sind, gemäß Anspruch 3, wobei der ortsspezifischen Rekombinase erlaubt wird, vorübergehend zu wirken, indem ein ortsspezifisches Rekombinase-Gen, codierend die ortsspezifische Rekombinase, im Schritt (B) in die kultivierte Pflanzenzelle eingeführt wird.

6. Verfahren zum Herstellen einer Pflanzenzelle, in welcher ein oder mehrere Chromosomen verschwunden sind, gemäß Anspruch 5, wobei die Einführung des ortsspezifischen Rekombinase-Gens durch Einführen eines Vektors, umfassend ein ortsspezifisches Rekombinase-Gen und ein negativ selektierbares Marker-Gen, in eine Pflanzenzelle ausgeführt wird.

7. Verfahren zum Herstellen einer Pflanzenzelle, in welcher ein oder mehrere Chromosomen verschwunden sind, gemäß Anspruch 5,
wobei das ortsspezifische Rekombinase-Gen zwischen den zwei ortsspezifischen Rekombinase-Erkennungssequenzen, orientiert in der gleichen Richtung, welche durch die ortsspezifische Rekombinase, codiert durch das ortsspezifische Rekombinase-Gen, erkannt werden, in einem Vektor positioniert ist, und
der Vektor in eine Pflanzenzelle eingeführt wird.

8. Verfahren zum Herstellen einer Pflanzenzelle, in welcher ein oder mehrere Chromosomen verschwunden sind, gemäß Anspruch 7, wobei ein negativ selektierbares Marker-Gen, zusammen mit einem ortsspezifischen Rekombinase-Gen, zwischen den zwei ortsspezifischen Rekombinase-Erkennungssequenzen, orientiert in der gleichen Richtung, in dem Vektor positioniert ist.

9. Verfahren zum Herstellen einer Pflanzenzelle, in welcher ein oder mehrere Chromosomen verschwunden sind, gemäß einem der Ansprüche 1 bis 3, 6 und 8, wobei ein letales Induktionsgen oder ein Induktionsgen für eine morphologische Abnormalität als das negativ selektierbare Marker-Gen verwendet wird.

## Revendications

1. Procédé de production d'une cellule végétale dans laquelle un ou plusieurs chromosomes ont disparu, ledit procédé comprenant les étapes (A)-(C) suivantes:
(A) introduire un vecteur comprenant:
deux séquences de reconnaissance de recombinase site-spécifique orientées dans la direction opposée,
un gène de recombinase site-spécifique qui est positionné à l'intérieur ou à l'extérieur de la région entre les deux séquences de reconnaissance de recombinase site-spécifiques et qui code une recombinase site-spécifique qui reconnaît les deux séquences de reconnaissance de recombinase site-spécifique, et
un gène marqueur sélectionnable négatif positionné à l'intérieur ou à l'extérieur de la région entre les deux séquences de reconnaissance de recombinase site-spécifique orientées dans la direction opposée,
dans une cellule végétale;
(B) cultiver et faire croître la cellule végétale à vecteur introduit (A); et
(C) sélectionner une cellule dans laquelle un chromosome a disparu, de la cellule cultivée dans (B),
dans lequel la cellule végétale est cultivée dans des conditions dans lesquelles le gène marqueur sélectionnable négatif fonctionne à l'étape (B).

2. Procédé de production d'une cellule végétale dans laquelle un ou plusieurs chromosomes ont disparu, ledit procédé comprenant les étapes (A)-(C) suivantes:
(A) introduire un vecteur comprenant:
une séquence de reconnaissance de recombinase site-spécifique comprenant une séquence de nucléotides à symétrie ponctuelle,
un gène de recombinase site-spécifique codant une recombinase site-spécifique qui reconnaît la séquence de reconnaissance de recombinase site-spécifique, et
un gène marqueur sélectionnable négatif,
dans une cellule végétale;
(B) cultiver et faire croître la cellule végétale à vecteur introduit dans (A); et
(C) sélectionner une cellule dans laquelle un chromosome a disparu, de la cellule cultivée dans (B).
dans lequel la cellule végétale est cultivée dans des conditions dans lesquelles le gène marqueur sélectionnable fonctionne à l'étape (B).

3. Procédé de production d'une cellule végétale dans laquelle un ou plusieurs chromosomes ont disparu, ledit procédé comprend les étapes (A)-(D) suivantes:
(A) introduire
un vecteur comprenant deux séquences de reconnaissance de recombinase site-spécifique orientées dans la direction opposée et un gène marqueur sélectionnable négatif positionné à l'intérieur ou à l'extérieur de la région entre les deux séquences de reconnaissance de recombinase site-spécifique, ou
un vecteur comprenant une séquence de reconnaissance de recombinase site-spécifique comprenant une séquence de nucléotides à symétrie ponctuelle et un gène marqueur sélectionnable négatif,
dans une cellule végétale;
(B) cultiver la cellule végétale à vecteur introduit dans (A) et permettre à la recombinase site-spécifique qui reconnaît les deux séquences de reconnaissance de recombinase site-spécifique d'agir passagèrement dans la cellule pendant au moins la croissance de la cellule;
(C) cultiver et faire croître une cellule dans laquelle la recombinase site-spécifique a été autorisée à agir dans (B); et
(D) sélectionner une cellule dans laquelle un chromosome a disparu, de la cellule cultivée dans (C),
dans lequel la cellule végétale est cultivée dans des conditions dans lesquelles le gène marqueur sélectionnable négatif fonctionne à l'étape (C).

4. Procédé de production d'une cellule végétale dans laquelle un ou plusieurs chromosomes ont disparu selon la revendication 3, dans lequel la recombinase site-spécifique est autorisée à agir passagèrement en introduisant la recombinase site-spécifique dans la cellule végétale cultivée à l'étape (B).

5. Procédé de production d'une cellule végétale dans laquelle un ou plusieurs chromosomes ont disparu selon la revendication 3, dans lequel la recombinase site-spécifique est autorisée à agir passagèrement en introduisant un gène de recombinase site-spécifique codant la recombinase site-spécifique dans la cellule végétale cultivée à l'étape (B).

6. Procédé de production d'une cellule végétale dans laquelle un ou plusieurs chromosomes ont disparu selon la revendication 5, dans lequel l'introduction du gène de recombinase site-spécifique est effectuée en introduisant un vecteur comprenant un gène de recombinase site-spécifique et un gène marqueur sélectionnable négatif, dans une cellule végétale.

7. Procédé de production d'une cellule végétale dans laquelle un ou plusieurs chromosomes ont disparu selon la revendication 5,
dans lequel le gène de recombinase site-spécifique est positionné entre les deux séquences de reconnaissance de recombinase site-spécifique orientées dans la même direction, lesquelles sont reconnues par la recombinase site-spécifique codée par le gène de recombinase site-spécifique, dans un vecteur, et
le vecteur est introduit dans une cellule végétale.

8. Procédé de production d'une cellule végétale dans laquelle un ou plusieurs chromosomes ont disparu selon la revendication 7, dans lequel un gène marqueur sélectionnable négatif, ensemble avec un gène de recombinase site-spécifique, est positionné entre les deux séquences de reconnaissance de recombinase site-spécifique orientées dans la même direction, dans le vecteur.

9. Procédé de production d'une cellule végétale dans laquelle un ou plusieurs chromosomes ont disparu selon l'une quelconque des revendications 1 à 3, 6 et 8, dans lequel un gène d'induction létale ou un gène d'induction d'anomalie morphologique est utilisé en tant que gène marqueur sélectionnable négatif.


### SEQUENCE LISTING

<110> Nippon Paper Industries Co., Ltd.
<120> The method for producing the plant cell that lost at least one chromosomes
<130> P05610100
<150> JP 2004-382394
   <151> 2004-12-08
<150> JP 2005-174225
   <151> 2005-05-18
<160> 20
<210> 1
   <211> 28
   <212> DNA
   <213> Artificial Sequence
<220>
   <223> primer for PCR
<400> 1
   gctctagagc atgtggaggc taacagtg 28
<210> 2
   <211> 28
   <212> DNA
   <213> Artificial Sequence
<220>
   <223> primer for PCR
<400> 2
   gcgagctctc agtgctctac gtaggccg 28
<210> 3
   <211> 139
   <212> DNA
   <213> Artificial Sequence
<220>
   <223> oligo nucleotide for PCR marker
<400> 3
   tgcagaataa ataaacgcca tggcacccgg gaaagaaata aaatgaaacg caaacacatg 60
   acctgcaggc acataagatg atacgcaagc gccgagctct actacgcaat ggcagacgca 120
   aaagcaggct acgcatgca 139
<210> 4
   <211> 139
   <212> DNA
   <213> Artificial Sequence
<220> .
   <223> oligo nucleotide for PCR marker
<400> 4
   tgcatgcgta gcctgctttt gcgtctgcca ttgcgtagta gagctcggcg cttgcgtatc 60
   atcttatgtg cctgcaggtc atgtgtttgc gtttcatttt atttctttcc cgggtgccat 120
   ggcgtttatt tattctgca 139
<210> 5
   <211> 18
   <212> DNA
   <213> Artificial Sequence
<220>
   <223> oligo nucleotide for vector construction
<400> 5
   ctgcacgcta cctgcagg 18
<210> 6
   <211> 18
   <212> DNA
   <213> Artificial Sequence
<220>
   <223> oligo nucleotide for vector construction
<400> 6
   cctgcaggta gcgtgcag 18
<210> 7
   <211> 20
   <212> DNA
   <213> Artificial Sequence
<220>
   <223> primer for PCR
<400> 7
   agaggctatt cggctatgca 20
<210> 8
   <211> 20
   <212> DNA
   <213> Artificial Sequence
<220>
   <223> primer for PCR
<400> 8
   ccatgatatt cggcaagcag 20
<210> 9
   <211> 25
   <212> DNA
   <213> Artificial Sequence
<220>
   <223> primer for PCR
<400> 9
   actgatagtt taaactgaag gcggg 25
<210> 10
   <211> 20
   <212> DNA
   <213> Artificial Sequence
<220>
   <223> primer for PCR
<400> 10
   atggcgttta tttattctgc 20
<210> 11
   <211> 20
   <212> DNA
   <213> Artificial Sequence
<220>
   <223> primer for PCR
<400> 11
   acataagatg atacgcaagc 20
<210> 12
   <211> 22
   <212> DNA
   <213> Artificial Sequence
<220>
   <223> primer for PCR
<400> 12
   aagccggcga acgtggcgag aa 22
<210> 13
   <211> 20
   <212> DNA
   <213> Artificial Sequence
<220>
   <223> primer for PCR
<400> 13
   cgtctgtcga gaagtttctg 20
<210> 14
   <211> 20
   <212> DNA
   <213> Artificial Sequence
<220>
   <223> primer for PCR
<400> 14
   ctatcggcga gtacttctac 20
<210> 15
   <211> 25
   <212> DNA
   <213> Artificial Sequence
<220>
   <223> primer for PCR
<400> 15
   aaacgacaat ctgatcatga gcgga 25
<210> 16
   <211> 24
   <212> DNA
   <213> Artificial Sequence
<220>
   <223> primer for PCR
<400> 16
   ggctcgtatg ttgtgtggaa ttgt 24
<210> 17
   <211> 25
   <212> DNA
   <213> Artificial Sequence
<220>
   <223> primer for PCR
<400> 17
   ccaaggatac tgaaatttca acaat 25
<210> 18
   <211> 25
   <212> DNA
   <213> Artificial Sequence
<220>
   <223> primer for PCR
<400> 18
   ttatttgaaa gatatgaagc tgtca 25
<210> 19
   <211> 25
   <212> DNA
   <213> Artificial Sequence
<220>
   <223> primer for PCR
<400> 19
   taagacctat atccacttca aaaca 25
<210> 20
   <211> 25
   <212> DNA
   <213> Artificial Sequence
<220>
   <223> primer for PCR
<400> 20
   agctagccct gtggcgtgcc ttcca 25